# EUROPEAN PATENT APPLICATION

(11) **EP 1 508 619 A1**
(43) Date of publication of application: **23.02.2005**
(21) Application number: 03733501.5
(22) Date of filing: 19.06.2003
(51) Int. Cl.: C12N 15/11, C12N 1/15, C12N 1/19, C12N 1/21, C12N 5/10, A61P 13/12

(54) **NOVEL PROMOTER**

(30) Priority: 20.06.2002 JP 2002180543
(71) Applicant: YAMANOUCHI PHARMACEUTICAL CO. LTD., Tokyo 103-8411 (JP)
(72) Inventor: TAKEDA, Masayoshi, /o Yamanouchi Pharm. Co., Ltd, Tsukuba-shi, Ibaraki 305-8585 (JP); ABE, Kunitake, c/o Yamanouchi Pharm. Co., Ltd, Tsukuba-shi, Ibaraki 305-8585 (JP); YAMAJI, Noboru, c/o Yamanouchi Pharm. Co., Ltd, Tsukuba-shi, Ibaraki 305-8585 (JP)
(74) Representative: Bates, Philip Ian
(86) International application number: PCT/JP2003/007807
(87) International publication number: WO 2004/001047

(57) **Abstract**

Disclosing a promoter of the protease MDTS9, which is induced by TGF-β, which is involved in the metabolism of extracellular matrix, and in which the expression of the gene thereof is elevated in renal failure model animals, which promoter is useful as a tool for screening an agent for treating and/or preventing chronic renal failure, as well as a method for screening an agent for treating and/or preventing chronic renal failure by selecting an inhibitor of the promoter.

## Description

### TECHNICAL FIELD

The present invention relates to a novel promoter.

### BACKGROUND ART

ADAMTS (a disintegrin and metalloprotease with thrombospondin motifs) is a group of molecules containing a disintegrin-like domain, a metalloprotease-like domain, and a thrombospondin type I repeat sequence (referred to as TSP-1 repeat sequence hereinafter).

Among the human ADAMTS molecules, it has been indicated that ADAMTS4 (aggrecanase-1) and ADAMTS11 (aggrecanase-2) have an activity to selectively cleave an extracellular matrix aggrecan and additionally that these enzymes may possibly be enzymes responsible for the degradation of the cartilage extracellular matrix aggrecan in arthritis or osteoarthritis (Tortorella M. D., *et al., Science,* **284**, 1664-1666, 1999; and Abbaszade I., *et al., J. Biol. Chem*., **274**, 23443-23450, 1999). Further, it has been shown that ADAMTS2 (procollagen I N-proteinase) is involved as an enzyme cleaving and removing the N-terminal region of type I procollagen in the conversion of type I procollagen to the mature type, so ADAMTS2 plays an important role in the formation of collagen fiber, and that an abnormality in the gene thereof has relationship with type VIIC Ehlers-Danlos syndrome (Colige A., *et al., Am. J. Hum. Genet.,* **65**, 308-317, 1999).

In other words, it has been indicated that ADAMTS molecules are involved in the metabolism of extracellular matrices such as aggrecan and collagen, including the degradation and maturation thereof.

Meanwhile, chronic renal failure is a disease with characteristics of glomerulosclerosis and renal interstitial fibrosis. It is believed that the qualitative change and/or quantitative increase of extracellular matrix components is the main mechanism of the onset and progress of the disease. It has been known that transforming growth factor β (TGF-β) is a differentiation and growth factor with a wide range of various physiological functions. It has also been known that transforming growth factor β (TGF-β) has an activity responsible for the qualitative change and quantitative increase of components of extracellular matrix (Border W. A., *et al., N. Engl. J. Med.,* **331**, 1286-1292, 1996; Roberts, A. B., *et al., Proc. Natl. Acad. Sci. U*.*S*.*A*., **83**, 4167-4171, 1986; Fukabori, Y., *et al., Int. J. Urol.,* **4**, 597-602, 1997; Lohr, M., *et al., Cancer Res.,* **61**, 550-555, 2001). Since it was shown that in an experiment using a renal failure model, the introduction of the gene of decorin, a protein suppressing the specific action of TGF-β (Isaka Y., *et al., Nature Med.,* **2**, 418-423, 1996) and the administration of anti-TGF-β (Ziyadeh F.N., *et al., Proc. Natl. Acad. Sci. U.S.A.*, **97**, 8015-8020, 2000; Sharma K., *et al., Diabetes,* **45**, 522-530, 1996; and Border W.A. *et al*., Nature, **346**, 371-374, 1990) were effective, it is considered that the suppression or inhibition of the physiological functions of TGF-β can lead to the treatment of chronic renal failure.

Under such current circumstance with no satisfactory agent for treating chronic renal failure, no TGF-β inhibitor has been introduced into market so far, although the expectation toward such agent is very high.

Additionally, the mechanism of interstitial inflammation consequently causing fibrosis has been examined intensively. In kidney, inflammatory cytokines such as IL-1β generated and released in glomerulus activate the epithelial cell of renal tubule to generate chemokines such as MCP-1, cell-adhesion molecules and extracellular matrices such as integrin and osteopontin to promote cellular invasion, so that the monocytes and T lymphocytes in invasion additionally secrete fresh cytokines, to affect epithelial ureter cells and the invaded cells to generate an inflammatory lesion, leading to a tissue destruction involving the degradation of extracellular matrix components. It is considered that the qualitative change and quantitative increase of extracellular matrix components due to excess repair of damaged tissues in the course of repairing the destroyed tissues and due to the involvement of fibroblasts and myofibroblasts would be fibrosis mechanism [Koichi Okada, Bessatu *Igaku no Ayumi,* Shikyutai *Jin-en no Hassho to Chiryo* (Medical Progress, Supplementary Edition, Onset and Therapeutic Treatment of Glomerular Nephritis), 120-123, 2001].

The NCBI database discloses the sequence of Homo sapiens chromosome 5 clone CTC-485121 of 187084 bp as AC010269 (see Non-patent Reference 1). The cDNA encoding a protein which presumably belongs to the ADAMTS family based on the analysis of the homology to known sequences and is known to have features such as high expression in ovary, the variation of the expression depending on the sexual cycle and the reduction of the protein existing in tumor cells has been known, together with a putative amino acid sequence thereof (see Patent Reference 1). Additionally, the nucleotide sequences possibly encoding proteases comprising sequences presumably belonging to the ADAM family and putative amino acid sequences encoded thereby, by using a genome database (see Patent Reference 2) have been known.
Non-patent Reference 1
http://www.ncbi.nlm.nih.gov/entrez/query.fcgi?cmd=Retrieve& db=nucleotide&list_uids=15281193&dopt=GenBank AC010269
Patent Reference 1: Pamphlet of International Publication WO 02/31163
Patent Reference 2: Pamphlet of International Publication WO 01/83782

### DISCLOSURE OF INVENTION

Because TGF-β is a differentiation and growth factor with a wide variety of physiological functions, inhibition of all the physiological functions of TGF-β may cause problem in the treatment of chronic renal failure possibly including long-term pharmaceutical administration. It is preferable to suppress or inhibit part of the physiological functions of TGF-β, which are involved in the qualitative change and quantitative increase of extracellular matrix components.

An object of the present invention is to provide a novel promoter for a protease which is induced by TGF-β and involved in the metabolism of extracellular matrices, which promoter is useful as a tool for screening an agent for treating and/or preventing chronic renal failure.

With the aim of solving the aforementioned problems, the inventors of the present invention made investigations. As a result, a polynucleotide encoding a novel protease MDTS9 consisting of a sequence of the first to 1224th amino acids of SEQ ID NO: 2 was obtained. Then, a promoter for MDTS9 from human genome DNA was obtained. Further, the inventor confirmed those described below. (1) Because the protease is classified as an ADAMTS protease, it is considered that the protease is a protease involved in the metabolism of extracellular matrices. (2) It is observed that the protease is actually expressed in human kidney. (3) It was found that the expression of the gene of the protease was increased in a renal failure model animal. (4) Further, it was confirmed that the activity of the promoter was enhanced with TGF-β (i.e., the expression of the protease was induced). These revealed that MDTS9 is a polypeptide which causes renal failure; and that screening a substance useful as an agent for treating and/or preventing chronic renal failure which suppresses or inhibits only some of the physiological functions of TGF-β that are involved in the qualitative change and quantitative increase of extracellular matrix components can be carried out by screening a substance suppressing the MDTS9 expression using the promoter for the polypeptide. Additionally, the inventors of the present invention found that inflammatory cytokine IL-1 inducing the expression of degradation enzymes of extracellular matrices, such as matrix metalloprotease (MMP), reduces the activity of the promoter of the present invention (i.e., suppresses the protease expression), and that the induction of the protease expression was specific not to the period of inflammation but to the course of tissue repairing and renal fibrosis with a feature of the qualitative change and quantitative increase of extracellular matrix components. Thus, the inventors of the present invention showed that inhibitors of the promoter of the present invention are useful as an agent for treating and/or preventing chronic renal failure. Thus, the present invention has been achieved.

Accordingly, the present invention relates to:
(1) A polynucleotide having a promoter activity for the polypeptide of SQ ID NO:2 and comprising a nucleotide sequence consisting of a 3253rd to 5023rd nucleotides in SEQ ID NO:17 or a nucleotide sequence consisting of the 3253rd to the 5023rd nucleotides in SEQ ID NO:17 in which 1 to 10 nucleotides are substituted, deleted, and/or inserted.
(2) A polynucleotide according to (1), comprising a nucleotide sequence of SEQ ID NO:17 or a nucleotide sequence consisting of a nucleotide sequence of SEQ ID NO:17 in which 1 to 10 nucleotides are substituted, deleted, and/or inserted.
(3) A polynucleotide according to (1), comprising the nucleotide sequence consisting of the 3253rd to 5023rd nucleotides in SEQ ID NO:17 or the nucleotide sequence of SEQ ID NO:17.
(4) An expression vector comprising the polynucleotide according to any one of (1) to (3).
(5) A cell transfected with the expression vector according to (4).
(6) A screening tool for a substance for the treatment and/or prevention of chronic renal failure, comprising the polynucleotide according to (1) or the cell according to (5).
(7) Use of the polynucleotide according to (1) or the cell according to (5) for screening a substance for the treatment and/or prevention of chronic renal failure.
(8) A method for analyzing a test compound about whether or not the test compound inhibits the promoter activity of the polynucleotide according to any one of (1) to (3), comprising (i) a step of allowing the test compound to contact with the cell according to claim 5 and (ii) a step of detecting the promoter activity.
(9) A method for screening a substance suppressing the expression of a polypeptide of SEQ ID NO:2, comprising the analyzing step by the method according to (8) and a step of selecting a substance inhibiting the promoter activity.
(10) A method for screening a substance for the treatment and/or prevention of chronic renal failure by the method according to (9).
(11) A method for producing a pharmaceutical composition for the treatment and/or prevention of chronic renal failure, comprising the analyzing step by the method according to (8) and a formulation step.

AC010269 discloses a sequence containing the nucleotide sequence of SEQ ID NO:17 as a part of the 187084 bp sequence. However, if the 187084-bp sequence is introduced into the upstream of the MDTS9 coding sequence the MDTS9 promoter activity is not supposed to be exhibited. Additionally, there is no disclosure about that the sequence has the promoter activity for MDTS9 or about screening an agent for treating and/or preventing chronic renal failure. In WO 02/31163, a sequence which differs by one amino acid from the amino acid sequence of SEQ ID NO:2 is described and it is indicated that the polypeptide of the sequence is involved in the degradation and reconstruction of the extracellular matrix in ovary. Additionally, in WO 01/83782, a sequence which differs by two amino acids from the amino acid sequence of SEQ ID NO:2 is described, however, it is not described that the polypeptide of the sequence was obtained or the activity or function thereof was confirmed. Further, there is no disclosure or suggestion about the polypeptides of the sequences described in the two publications as the etiology of chronic renal failure, about a promoter for MDTS9 or about screening a treating and/or preventing agent for chronic renal failure using a promoter for MDTS9.

### Brief Description of the Drawings

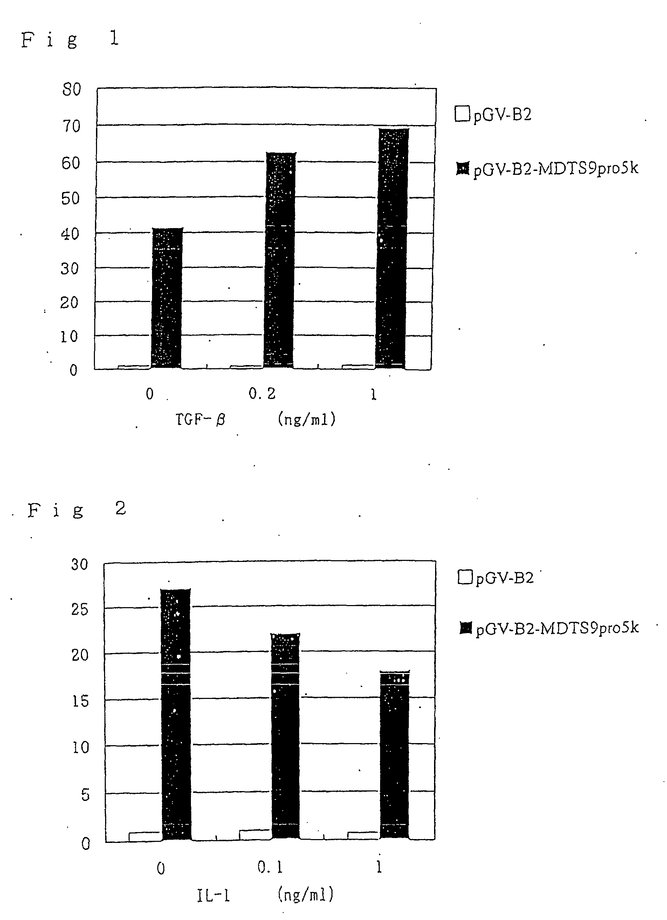

Fig. 1 shows the increase of luciferase activity in a cell in which pGV-B2-MDTS9pro5k is introduced according to TGF-β addition.

In the graphs, the vertical axis indicates the assay value of luciferase activity, after the assay value is corrected on the basis of the activity value of β-gal expressed with a β-gal expression plasmid simultaneously introduced therein and is then indicated as the relative value to the corrected value of the cell which is never treated with TGF-β and in which pGV-B2 (blank vector) is introduced as defined to be 1, while the horizontal axis indicates the concentration of TGF-β.

Fig. 2 shows the decrease of luciferase activity in a cell in which pGV-B2-MDTS9pro5k introduced according to IL-1 addition.

In the graphs, the vertical axis indicates the assay value of luciferase activity, after the assay value is corrected on the basis of the activity value of β-gal expressed with a β-gal expression plasmid simultaneously introduced therein and is then indicated as the relative value to the corrected value of the cell which is never treated with IL-1 and in which pGV-B2 (blank vector) is introduced as defined to be 1, while the horizontal axis indicates the concentration of IL-1.

### BEST MODE FOR CARRYING OUT THE INVENTION

The present invention will be explained in detail hereinafter.

### (1) The polynucleotide as the promoter of the present invention, expression vector and cell therefor

The inventors of the present invention obtained a polynucleotide encoding MDTS9 (metalloprotease and disintegrin with thrombospondin type-1 repeats 9) which is a protease involved in the metabolism of extracellular matrix and is observed to be expressed in human kidney and of which the expression of the gene is increased in a renal failure model animal, as well as a promoter for the protease, to determine the nucleotide sequence (SEQ ID NO:17). The obtained full-length promoter and partial fragments thereof (the 3253rd to 5023rd nucleotides in SEQ ID NO: 17) were subcloned in an appropriate plasmid, specifically a pGV-B2 plasmid containing the luciferase gene as a reporter gene. By detecting the expression of the reporter gene in the fusion plasmid, i.e. detecting luciferase expression based on the activity, it was confirmed that the obtained polynucleotide had the promoter activity. Additionally, the inventors of the present invention found that the promoter activity of the polynucleotide of SEQ ID NO:17 increased with TGF-β and decreased with Il-1.

Because the substance suppressing MDTS9 expression (inhibiting the promoter) is useful as an agent for treating and/or preventing chronic renal failure, as described above, the promoter of the present invention is useful for screening such inhibitor. So as to screen an inhibitor of the MDTS9 promoter, a sequence including the TGF-β response sequence in SEQ ID NO:17 is not essentially used. For example, screening by the method described in Example 7 is possible, by using the sequence consisting of the 3253rd to 5023rd nucleotides in SEQ ID NO:17. Thus, the promoter of the present invention may contain any nucleotide sequence in the 5' region as long as the promoter contains the nucleotide sequence of the 3253rd to 5023rd nucleotides in SEQ ID NO:17 and has the promoter activity for MDTS9. More preferably, the promoter of the present invention is a polynucleotide containing the nucleotide sequence in SEQ ID NO:17 and having the promoter activity for MDTS9. Still more preferably, the promoter is the polynucleotide of the nucleotide sequence of SEQ ID NO:17. The promoter of the present invention furthermore includes a polynucleotide comprising a nucleotide sequence in which one to plural nucleotides (preferably 1 to 10, more preferably 1 to 5, still more preferably 1 to 3) are substituted, deleted and/or inserted at any 1 to 10 sites (preferably 1 to 5, more preferably 1 to 3) in the nucleotide sequence of the 3253rd to 5023rd nucleotides in SEQ ID NO:17 and with the promoter activity for MDTS9 and more preferably a polynucleotide of a nucleotide sequence in which one to plural nucleotides (preferably 1 to 10, more preferably 1 to 5, still more preferably 1 to 3) are substituted, deleted and/or inserted at any one to 10 sites (preferably 1 to 5, more preferably 1 to 3) in the nucleotide sequence of SEQ ID NO:17 and with the promoter activity for MDTS9. Herein, the term "promoter activity" means an activity functioning as an initiation site of transcribing the information of DNA chain on RNA chain. The term "with the promoter activity for MDTS9" means that the promoter activity can be confirmed by the method described in Example 2, more preferably means that the promoter activity is elevated with TGF-β by the method described in Example 3.

The polynucleotide of the present invention may be produced by the following methods other than the methods described in the Examples.

### 1. Production by PCR

As described in Example 1, DNA containing the nucleotide sequence of SEQ ID NO:17 can be produced by carrying out PCR using primer and human genome DNA. Generally, the presence of allele mutant is well known. DNA comprising a nucleotide sequence in which nucleotides are substituted, deleted and/or inserted at any sites in the nucleotide sequence of SEQ ID NO:17 and having the MDTS9 promoter activity may be obtained in some case of the DNA production by the present method. Such DNA is also included in the promoter of the present invention.

### 2. Production using DNA synthesis

The polynucleotide can be produced by dividing the sequence of SEQ ID NO:17 and the complimentary chain thereof into several chains, then preparing the chains by chemical synthesis process, and combining the resulting DNA fragments together. Individual DNA fragments can be synthetically prepared by using DNA synthesizers (for example, Oligo 1000M DNA Synthesizer (Beckman) or 394 DNA/RNA Synthesizer (Applied Biosystems)).

Whether or not the produced polynucleotide has the promoter activity for MDTS9 can be confirmed for example by the method described in Example 2 or 3.

A person skilled in the art can prepare a polynucleotide with the same promoter activity as that of the natural promoter polynucleotide by modifications of a part of the nucleotide sequence of the promoter sequence of the natural type, such as substitution with other nucleotides or deletion of nucleotides and/or addition of nucleotides. As described above, a polynucleotide comprising a nucleotide sequence in which the natural nucleotide sequence are substituted, deleted and/or added and with the same promoter activity as that of the natural promoter polynucleotide is also included in the polynucleotide of the present invention. Nucleotides can be modified by methods by deletion introduction with restriction enzymes or DNA exonuclease, introduction of mutation by site-directed mutagenesis [*Nucleic Acid Res.* **10**, 6487 (1982)], modification of promoter sequence by PCR using mutant primer and direct introduction of synthetically mutated DNA (Maniatis, T. *et al*. (1989): "*Molecular Cloning - A Laboratory Manual* 2^{nd} Edt.", Cold Spring Harbor Laboratory, NY).

The polynucleotide as the MDTS9 promoter of the present invention can be used for the treatment and prevention of diseases due to deletion or abnormal expression of the MDTS9 protein as caused by mutations in biological organisms. For example, the MDTS9 gene including the MDTS9 promoter and the MDTS9 coding region of the present invention is inserted in vectors such as retrovirus, adenovirus and adeno-associated virus or is included in liposome for introduction into somatic cells, to express the MDTS9 protein under normal transcription controls, thereby enabling the amelioration of diseases due to deletion or abnormal expression of the MDTS9 protein.

DNA containing at least a part of the DNA of SEQ ID NO:17 can inhibit the binding between the MDTS9 promoter of the present invention and a protein capable of binding thereto (for example, transcription factor) in an antagonistic manner, despite the presence or absence of the promoter activity in the DNA. Therefore, in case that the DNA corresponds to the binding site of the protein inhibiting the activity of the promoter of the present invention, the administration of the DNA can promote the promoter activity, while in case that the DNA corresponds to the binding site of a protein promoting the promoter activity, the administration of the DNA can inhibit the promoter activity. The DNA for use in the antagonistic inhibition has a chain length of generally at least 6 nucleotides or more, preferably 10 nucleotides or more.

The recombinant vector of the present invention can be produced by integrating the polynucleotide of the present invention into a vector appropriately selected in accordance with the purpose. For example, in case of intending to construct a screening system for a substance regulating the MDTS9 promoter activity, preferably, the polynucleotide of the present invention is integrated into a vector in which a reporter gene of such as luciferase is integrated to produce the recombinant vector as described in Examples. Additionally, for the purpose of the gene therapy of diseases due to the deletion or abnormal expression of the MDTS9 protein, the promoter of the present invention and DNA of the MDTS9 coding region are integrated in a vector such as retrovirus, adenovirus and adeno-associated virus to produce the recombinant vector.

The transformant of the present invention can be produced by introducing the polynucleotide of the present invention in a host cell appropriately selected in accordance with the purpose. For example, in case of intending to construct, a screening system for a substance regulating the MDTS9 promoter activity, a cell derived from humans or mammalian animals such as mouse and rat and additionally from their renal tissues is preferably used.

### (2) Analysis method and screening method in of the present invention

By using the promoter of the present invention, it can be analyzed whether or not a test compound inhibits the MDTS9 promoter activity. Additionally, when the analysis method of the present invention is used, a substance inhibiting the MDTS9 promoter activity can be screened. Because it is believed that MDTS9 is an ADAMTS protease on the basis of the sequence, MDTS9 is supposed to be a protease involved in the metabolism of extracellular matrix. As shown in Reference Examples 5 and 7 described below, the protease is a protein expressed in human kidney. As shown in Reference Example 6, the expression of the gene is elevated in a renal failure model animal. As shown in Reference Example 3 below, additionally, MDTS9 is an ADAMTS protease, of which the expression is induced by TGF-β and is suppressed by IL-1. Therefore, the substance inhibiting the activity of the promoter of the present invention highly possibly suppresses or inhibits just some of the physiological functions of TGF-β, which are involved in the qualitative change and quantitative increase of the extracellular matrix, so that such substance is useful as the active component of an agent for treating chronic renal failure possibly including long-term pharmaceutical administration. Thus, a cell expressing the promoter of the present invention can be used as a screening tool for the substance inhibiting the activity of the promoter of the present invention or an agent for treating and/or preventing chronic renal failure.

The test compounds applicable to the analysis method or screening method of the present invention are not particularly limited and include for example, various known compounds (including peptides) registered in the Chemical File, compound groups obtained by the combinatorial chemistry technique (Terrett, N. K., *et al., Tetrahedron,* **51**, 8135-8137, 1995), or general synthetic techniques, or random peptide groups prepared by the application of the phage display method (Felici, F., *et al., J. Mol. Biol.,* **222**, 301-310, 1991). The known compounds described above include for example compounds (including peptides) which have known activities of inhibiting promoters but are still unknown as to whether or not the compounds inhibit the activity of the promoter of the present invention. Additionally, microbial culture supernatants, natural components from plants or marine organisms, or extracts from animal tissues may also be used as the test compound for screening. Further, compounds (including peptides) prepared by chemical or biological modification of the compounds (including peptides) selected by the screening method of the present invention may also be used.

The analysis method of the present invention includes a process of analyzing a test compound about whether or not the test compound inhibits the activity of the promoter of the present invention, including (i) a step of putting a cell transfected with an expression vector comprising the promoter of the present invention into contact with the test compound, and (ii) a step of detecting the activity of the promoter.

The method for detecting the activity of the promoter is not particularly limited and includes a method using a reporter gene plasmid carrying the sequence of SEQ ID NO:17 as described in Example 2 or 3, which can be carried out in a simple manner. The reporter gene means a gene encoding a protein which can be assayed by general methods (for example, assay methods known to a person skilled in the art, such as assaying enzyme activity). As such, genes of chloramphenicol acetyltransferase, luciferase, β-galactosidase and alkaline phosphatase are frequently used, although genes are not limited to those. Concerning the vector as a base for constructing the reporter gene plasmid, there is no limitation. Commercially available plasmid vectors for such as pGV-B2 (manufactured by Toyo Ink) and pSEAP2-Basic (manufactured by Clontech) can be used. The sequence is then inserted in the correct orientation upstream of the reporter genes of these vectors to prepare reporter gene plasmids. The amount of a reporter protein expressed in a cell transformed with such plasmid is assayed by a method appropriate for each of the reporter protein, to determine the presence or absence of the promoter activity of the sequence or the intensity thereof. By adding a test compound to a liquid culture of the transformed cell, the action of the test compound on the promoter activity can be analyzed.

Additionally, the present invention also includes a method for screening a substance suppressing the expression of the polypeptide (MDTS9) of SEQ ID NO:2, including (i) the analysis step described above and (ii) a step of selecting a substance inhibiting the promoter activity, or a method for screening an agent for treating and/or preventing chronic renal failure.

The substance inhibiting the promoter activity as selected by the screening method described above is preferably a substance at 90 % or less of the promoter activity in the case of TGF-β treatment alone, more preferably a substance at the same level or less as in the case of no TGF-β treatment, by a method described in Example 3 below.

### (3) Method for producing a pharmaceutical composition for treatment and/or prevention of chronic renal failure of the present invention

The present invention includes a method for producing a pharmaceutical composition for the treatment and/or prevention of chronic renal failure, including an analysis step according to the analysis method as described in (2) and a formulation step of the present invention. The method for producing a pharmaceutical composition for the treatment and/or prevention of chronic renal failure also includes a method for producing a pharmaceutical composition for the treatment and/or prevention of chronic renal failure including a step of analyzing the presence or absence of the inhibition of the activity of the protease of the present invention by the analysis method of the present invention at a confirmation test according to the quality standard of pharmaceutical compositions for the therapeutic treatment and/or prophylaxis of chronic renal failure, and a formulation step.

Additionally, the present invention also includes a pharmaceutical composition for the treatment and/or prevention of chronic renal failure, which contains as the active ingredient, a substance inhibiting the activity of the protease of the present invention and selected by the screening method of the present invention as well as a method for the treatment and/or prevention of chronic renal failure including administration of a substance inhibiting the activity of the protease of the present invention. Then, the method for producing a pharmaceutical composition for the treatment and/or prevention of chronic renal failure of the invention also includes a method for producing a pharmaceutical composition for the treatment and/or prevention of chronic renal failure, which is a formulation step of the substance obtained by the screening method of the present invention including the analysis step of the invention as described in (2).

The formulation containing a substance inhibiting the activity of the protease of the present invention as the active ingredient can be prepared, using carriers, excipients and/or other additives for general use in preparing such formulation, depending on the type of the active ingredient.

The administration includes such as oral administration via tablets, pills, capsules, granules, fine granules, powders, oral solutions or the like, or parenteral administration via intravenous injections, intramuscular injections, or the like, suppositories, transdermal dosage forms, transmucosal dosage forms or the like. Particularly, for peptides to be digested in the stomach, a parenteral administration such as intravenous injections or the like, or formulation method for avoiding the digestion, such as the formulation measure described in the pamphlet of WO 95/28963 are preferably applied.

In the solid composition for oral administration, one or more active substances may be mixed with at least one inert diluent such as lactose, mannitol, glucose, microcrystalline cellulose, hydroxypropyl cellulose, starch, polyvinyl pyrrolidone or magnesium metasilicate aluminate. According to a general method, the composition described above may contain additives other than inert diluents, such as lubricants, disintegrators, stabilizers, or agents for solubilization or auxiliary agents for solubilization. If necessary, tablets or pills may be coated with a sugar coating or a film of a substance soluble in stomach or gut.

The liquid composition for oral administration may include for example emulsions, solutions, suspensions, syrups or elixirs, and may also contain an inert diluent for general use such as distilled water or ethanol. The composition may contain additives other than inert diluents, which are for example moistening agents, suspending agents, sweeteners, flavors, or antiseptics.

The injections for parenteral administration may include aqueous or non-aqueous solutions, suspensions, or emulsions, which are aseptic. Aqueous solutions or suspensions may contain distilled water for injections or physiological saline as a diluent for example. The diluent for use in the non-aqueous solutions and suspensions include such as propylene glycol, polyethylene glycol, plant oil (e.g., olive oil), alcohols (e.g., ethanol), and polysorbate 80. The composition may further contain a moistening agent, an emulsifier, a dispersant, a stabilizer, an agent for solubilization or an auxiliary agent for solubilization, an antiseptic or the like. The composition may be sterilized for example by filtration through a filter capable of retaining bacteria, blending a disinfectant, or irradiation. By producing a sterile solid composition and dissolving the composition in sterile water or other sterile media for injections prior to use, the resulting solution can be used.

Taking account of the intensity of the activity of the active ingredient selected by the aforementioned screening method, or the symptom, age, sex or the like of each patient to be administered, the dose is appropriately determined.

In case of oral administration, for example, the dose for an adult (60 kg in weight) is generally about 0.01 to 1,000 mg, preferably 0.01 to 100 mg per day. In case of parenteral administration in the form of an injection, the dose is about 0.01 to 1,000 mg, preferably 0.01 to 100 mg per day.

### EXAMPLES

The present invention will be further illustrated by, but is by no means limited to the following Examples. The Examples were carried out according to known methods (for example, Sambrook, J., *et al., "Molecular Cloning - A Laboratory Manual*", Cold Spring Harbor Laboratory, NY, 1989), unless otherwise stated.

### Reference Example 1: Preparation of expression vector of FLAG addition type at C terminus

Cleaving plasmid pCEP4 (manufactured by Invitrogen) with restriction enzymes *Cla*I and *Nsi*I and allowing the resulting plasmid to be blunt-ended, followed by self-ligation to prepare an expression vector pCEP4d, in which the EBNA1 expression unit derived from Epstein-Barr virus was deleted. The resulting expression vector pCEP4d was cleaved with restriction enzymes *Nhe*I and *Bam*HI, followed by agarose gel extraction, to obtain a DNA fragment of about 7.7 kbp. A double-stranded oligonucleotide prepared by annealing the oligonucleotide consisting of the nucleotide sequence of SEQ ID NO:3 and the oligonucleotide consisting of the nucleotide sequence of SEQ ID NO:4 together was then inserted into the resulting DNA fragment, to construct an expression vector pCEP4d-FLAG. It was confirmed that the resulting expression vector had the intended sequence on the basis of the nucleotide sequence.

Using the expression vector pCEP4d-FLAG as a template, a combination of the oligonucleotide consisting of the nucleotide sequence of SEQ ID NO:5 and the oligonucleotide consisting of the nucleotide sequence of SEQ ID NO:6 as primer, PCR was carried out with PyroBest DNA polymerase (PyroBest™; manufactured by Takara Shuzo Co., Ltd.). In the PCR, a thermal denaturing reaction was first performed at 94°C (2 minutes). Then, a cycle of treatments at 94°C (30 seconds), at 55°C (30 seconds) and at 72°C (30 seconds) was repeated 15 times. Finally, an extension reaction was carried out at 72°C (7 minutes). A DNA fragment of about 0.4 kbp obtained by the PCR was cleaved with a restriction enzyme *Spe*I, and was then inserted into the expression vector pCEP4d-FLAG (about 7.7 kbp) cleaved with *Xba*I, to obtain an expression vector pCEPdE2-FLAG. In the resulting expression vector pCEPdE2-FLAG, an *Xba*I recognition sequence, an *Nhe*I recognition sequence, a *Not*I recognition sequence, a *Bam*HI recognition sequence and the FLAG tag as cloning sites were arranged in this sequential order from the promoter toward the downstream.

### Reference Example 2: Cloning of full-length ORF gene of the gene of novel protease MDTS9

Using a combination of the oligonucleotide consisting of the nucleotide sequence of SEQ ID NO:7 (with a *Spe*I recognition sequence and a Kozak sequence which was added to the 5'side) and the oligonucleotide consisting of the nucleotide sequence of SEQ ID NO:8 (with a *Not*I recognition sequence which was added to the 5' side) as primers and the human fetal kidney cDNA library (Marathon-Ready™ cDNA; manufactured by Clontech) as a template, PCR was carried out with the DNA polymerase (TaKaRa LA Taq™; manufactured by Takara Shuzo Co., Ltd.). In the PCR, a thermal denaturing reaction was first performed at 94°C (2 minutes). Then, a cycle of treatments at 98°C (10 seconds) and 68°C (2 minutes and 30 seconds) was repeated 40 times. Finally, an extension reaction was carried out at 68°C (7 minutes). The DNA fragment of about 2.2 kbp obtained by the PCR (with the *Spe*I recognition sequence and the Kozak sequence which was added to the 5'terminus and with the *Not*I recognition sequence which was added to the 3' terminus) was subcloned into a plasmid PCR2.1 (manufactured by Invitrogen), to obtain a clone pMDTS9Cysl.

The resulting plasmid pMDTS9Cys1 was cleaved with restriction enzymes of *Spe*I and *Not*I, to obtain a DNA fragment of about 2.2 kbp, which was then inserted in between the *Xba*I site and *Not*I site of the plasmid pCEPdE2-FLAG constructed in Reference Example 1. In such manner, a plasmid pCEPdE2-MDTS9Cys1-FLAG was constructed.

Using a combination of the oligonucleotide consisting of the nucleotide sequence of SEQ ID NO:9 (with an *Spe*I recognition sequence and a Kozak sequence which was added to the 5' terminus) and the oligonucleotide consisting of the nucleotide sequence of SEQ ID NO:10 as primers and the human fetal kidney cDNA library (Marathon-Ready™ cDNA; manufactured by Clontech) as a template, PCR was carried out with the DNA polymerase (TaKaRa LA Taq™; manufactured by Takara Shuzo Co., Ltd.). In the PCR, a thermal denaturing reaction was first performed at 94°C (2 minutes). Then, a cycle of treatments at 98°C (10 seconds) and 68°C (30 seconds) was repeated 45 times. Finally, an extension reaction was carried out at 68°C (7 minutes). A DNA fragment of about 0.2 kbp (with the *Spe*I recognition sequence and the Kozak sequence which was added to the 5' terminus) obtained by the PCR was subcloned into the plasmid PCR2.1 (manufactured by Invitrogen), to obtain a clone pMDTS9(5S2-12). The inserted fragment is the DNA consisting of the nucleotide sequence of SEQ ID NO:1 and encodes a sequence of the first to 1224th amino acids in the amino acid sequence of SEQ ID NO:2.

The resulting plasmid pMDTS9(5S2-12) was cleaved with restriction enzymes of *Spe*I and *Nco*I, to generate a *Spe*I-*Nco*I DNA fragment A of about 0.2 kbp. The plasmid pMDTS9Cys1 obtained above was cleaved with restriction enzymes *Nco*I and *Not*I, to obtain an *Nco*I-*Not*I DNA fragment B of about 2.0 kbp. The DNA fragments A and B were inserted in between the *Xba*I site and *Not*I site of the pCEPdE2-FLAG constructed in Reference Example 1, to construct a plasmid pCEPdE2-MDTS9Cys2-FLAG. Similarly, the DNA fragment A and the DNA fragment B were inserted in between the *Spe*I site and *Not*I site of a plasmid pZErO-2 (manufactured by Invitrogen), to construct a plasmid pZErO-MDTS9Cys2.

Further, the plasmid pCEPdE2-MDTS9Cys2-FLAG carries a gene consisting of the 1st to 2250th nucleotides in the nucleotide sequence of SEQ ID NO: 1, which is the nucleotide sequence of the gene of the novel protease MDTS9 and can express in a host animal cell, in which a polypeptide of the amino acid sequence of SEQ ID NO:11 having been added to the C terminus of the sequence of the 1st to 750th amino acids in the amino acid sequence of SEQ ID NO:2. Herein, it is suggested that the polypeptide consisting of the amino acid sequence of SEQ ID NO:2 is the ADAMTS protease.

### Reference Example 3: Expression of truncated MDTS9 protein (MDTS9Cys2)

Using a commercially available transfection reagent (FuGENE™6 Transfection Reagent; manufactured by Boehringer Mannheim) and according to the protocol attached thereto, the plasmid pCEPdE2-MDTS9Cys2-FLAG prepared in Reference Example 2 or the plasmid pCEPdE2-FLAG prepared in Reference Example 1 as a control was introduced into HEK293-EBNA (manufactured by Invitrogen) cultured in a serum culture medium [DMEM (GIBCO-BRL), 10% fetal bovine serum, 100 µg/mL penicillin, 100 µg/mL streptomycin, and 250 µg/mL-G418 (manufactured by Nakarai Tesque, Inc.)].

After the introduction of each plasmid, the cells were cultured for 48 hours (referred to as serum culture hereinafter). After the introduction of each plasmid, otherwise, the cells were cultured for 16 hours, then washed twice with PBS, and cultured in a serum-free culture medium [DMEM (GIBCO-BRL), 100 µg/mL penicillin, 100 µg/mL streptomycin, and 250 µg/mL-G418 (manufactured by Nakarai Tesque, Inc.)] for 32 hours (referred to as serum-free culture hereinafter).

Each liquid culture obtained from the serum culture or the serum-free culture was centrifuged (at 3,000 rpm for 10 minutes) with a centrifuge (Type 8800; manufactured by Kubota Corporation), to obtain a culture supernatant. The individual cells remained after discarding the liquid culture were treated with an extraction solution [20 mmol/L HEPES (pH7.4), 1% Triton X-100, 1% glycerol, and 0.1% bovine serum albumin (BSA)] for 15 minutes. Subsequently, the cells were scraped from the culture plates by pipetting. The resulting cell suspension was centrifuged (at 3,000 rpm for 10 minutes) with a centrifuge (Type 8800; manufactured by Kubota Corporation), to separate a cell membrane-bound fraction (supernatant) from a cell fraction (pellet).

The expression of the intended protein in the resulting individual fractions (i.e., culture supernatant, cell membrane-bound fraction, and cell fraction) was confirmed by Western blotting using an antibody (mouse anti-FLAG monoclonal antibody M2; manufactured by Sigma) against the FLAG tag added to the C terminus. More specifically, each fraction as described above was electrophoresed on an SDS/10%-20% acrylamide gel (manufactured by Daiichi Pure Chemicals) under reductive conditions using 2-ME, and transferred to a polyvinylidene difluoride (PVDF) membrane with a blotting apparatus. To the resulting PVDF membrane after the transfer, a blocking agent (Block-ace manufactured by Dainippon Pharmaceutical) was added for blocking. Subsequently, the reaction sequentially with the mouse anti-FLAG monoclonal antibody M2 and a rabbit anti-mouse IgG polyclonal antibody labeled with horseradish peroxidase (manufactured by Zymed or TAGO) was carried out. After blocking, alternatively, the reaction sequentially with a biotinylated antibody M2 (manufactured by Sigma) and a streptoavidin labeled with horseradish peroxidase (manufactured by Amersham Pharmacia Biotech) was carried out. After the reaction, the expression of the intended protein was confirmed with a commercially available detection system by Western blotting (ECL Western Blotting Detection System; manufactured by Amersham Pharmacia Biotech).

The apparent molecular weight of the protein (i.e., truncated MDTS9 protein) in each fraction obtained from the serum-free culture of the cell which is introduced the plasmid pCEPdE2-MDTS9Cys2-FLAG as detected on the SDS-polyacrylamide gel electrophoresis (SDS-PAGE) was approximately 55 to 65 kDa in the culture supernatant, approximately 55 to 65 kDa in the cell membrane-bound fraction, and approximately 80 to 95 kDa in the cell fraction.

### Reference Example 4: Confirmation of protease activity of truncated MDTS9 protein

### (1) Construction of plasmid pCEPdE2-MDTS9Cys2E/Q-FLAG

Using Quick Change™ Site-Directed Mutagenesis Kit (manufactured by Stratagene) and according to the protocol attached thereto, a pZErO-MDTS9Cys2E/Q plasmid carrying a gene MDTS9Cys2E/Q was prepared. In the gene, Glu (glutamic acid) in the sequence of His-Glu-Ser-Gly-His (SEQ ID NO:12), which is recognized to be the activity center, was substituted with Gln (glutamine). As a template, the plasmid pZErO-MDTS9Cys2 prepared in Reference Example 2 was used, while as a primer set, the oligonucleotide consisting of the nucleotide sequence of SEQ ID NO:13 and the oligonucleotide consisting of the nucleotide sequence of SEQ ID NO:14 were used.

The resulting plasmid pZErO-MDTS9Cys2E/Q was cleaved with restriction enzymes *Spe*I and *Not*I, to generate a DNA fragment of about 2.3 kbp, which was then inserted in between the *Xba*I site and *Not*I site of the pCEPdE2-FLAG plasmid constructed in Reference Example 1, to obtain a pCEPdE2-MDTS9Cys2E/Q-FLAG plasmid.

### (2) Confirmation of protease activity, using complex formation with α₂-macroglobulin as marker

Each culture supernatant from the serum cultures of individual cells transfected with the pCEPdE2-MDTS9Cys2-FLAG plasmid prepared in Reference Example 2, the pCEPdE2-MDTS9Cys2E/Q-FLAG plasmid prepared in Reference Example 4(1), or the pCEPdE2-FLAG plasmid prepared in Reference Example 1 as a control, was electrophoresed (SDS-PAGE) under reductive conditions using 2-ME, and transferred to a PVDF membrane, by the same procedures as shown in Reference Example 3. To the resulting PVDF membrane after transfer, a blocking agent (Block-Ace manufactured by Dainippon Pharmaceutical) was added for blocking. Then, reaction sequentially with the goat anti-α₂-macroglobulin antibody (manufactured by CEDARLANE) and the rabbit anti-goat IgG polyclonal antibody labeled with horseradish peroxidase (manufactured by Zymed Laboratories) was carried out. After the reaction, the expression of the intended protein was confirmed with a commercially available detection system by Western blotting (ECL Western Blotting Detection System; manufactured by Amersham Pharmacia Biotech).

In the culture supernatant derived from the serum culture of the cell transfected with the pCEPdE2-MDTS9Cys2-FLAG plasmid, a band of about 250 kDa was detected, which was never detected in the culture supernatant derived from each serum culture of the cell transfected with the plasmid pCEPdE2-MDTS9Cys2E/Q-FLAG or the pCEPdE2-FLAG plasmid. This result reveals that the truncated MDTS9 protein (MDTS9Cys2) formed a complex with α₂-macroglobulin. Accordingly, it was confirmed that the truncated MDTS9 protein (MDTS9Cys2) had a protease activity.

### Reference Example 5: Confirmation of expression of MDTS9 gene in tissue distribution

A tissue distribution of the expression of the MDTS9 gene was analyzed by the following procedures, using commercially available cDNA panels [Human MTC Panel I (catalogue NO: K1420-1), Human MTC Panel II (catalogue NO: K1421-1), Human Fetal MTC Panel (catalogue NO: K1425-1), and Human Tumor MTC Panel (catalogue NO: K1422-1) in Multiple Tissue cDNA (MTC™) Panel manufactured by Clontech].

More specifically, PCR with DNA polymerase (TaKaRa LA Taq™; manufactured by Takara Shuzo Co., Ltd.) was carried out using a combination of the oligonucleotide consisting of the nucleotide sequence of SEQ ID NO:15 and the oligonucleotide consisting of the nucleotide sequence of SEQ ID NO:16 as primers and the cDNA panel described above as a template. In the PCR, a thermal denaturing reaction was first performed at 94°C (2 minutes). Then, a cycle of treatments at 98°C (10 seconds) and 68°C (1 minute and 30 seconds) was repeated 44 times. Then reaction solution was electrophoresed on an agarose gel, to detect a DNA fragment of about 1.1 kbp which is derived from the mRNA of the MDTS9 gene. As a result, it was indicated that the mRNA of the MDTS9 gene was expressed in kidney.

### Reference Example 6: Variation of expression of MDTS9 gene in renal failure model

### (1) Preparation of template cDNA

cDNA was prepared from the kidney of a rat 5/6 nephrectomy model [Kenjiro Kimura, "*Jin to Toseki* (*Kidney* *and Dialysis*)", 1991 (suppl.), 431-439]. On weeks 1, 2, 3, 4, 6, 8, and 10 after the completion of the 5/6 nephrectomy, 5 rats undergoned the 5/6 nephrectomy and 5 rats undergoned sham operation were autopsied to resect the kidneys. Immediately, the kidneys were frozen and kept at -80°C. The kidneys of each group were disrupted using a cell disrupter (CRYO-PRESS CP-100; manufactured by Microtec Nition) under freezing in liquid nitrogen. Subsequently, the total RNA was prepared using a reagent for purifying total RNA (ISOGEN; manufactured by Nippon Gene). The extracted total RNA was reacted with DNase (manufactured by Nippon Gene) at 37°C for 90 minutes. The DNase-treated total RNA of 0.25 µg was converted to cDNA by Superscript II First-Strand System (for RT-PCR; manufactured by GIBCO-BRL).

### (2) Variation of expression of MDTS9 gene in renal failure model in rat

For the analysis of the variation of the expression in the kidney of the renal failure model in rat, the cDNA prepared in Reference Example 6(1) was used as a template, together with a sequence detector (Prism7700 Sequence Detector; manufactured by Applied Biosystems). The oligonucleotide consisting of the nucleotide sequence of SEQ ID NO:30 and the oligonucleotide consisting of the nucleotide sequence of SEQ ID NO:31 were used as a primer set. Using a commercially available PCR reagent (SYBR Green PCR core reagent; manufactured by Applied Biosystems), PCR was carried out initially by denaturation at 95°C (10 minutes) and subsequently by repeating a cycle of treatments at 94°C (15 seconds), 60°C (30 seconds), and 72°C (60 seconds) 45 times.

In order to calculate the expression of the gene of human glyceraldehyde-3-phosphate dehydrogenase (G3PDH) as an internal standard, PCR was carried out under the same conditions, using the above cDNA as a template and the oligonucleotide consisting of the nucleotide sequence of SEQ ID NO:32 and the oligonucleotide consisting of the nucleotide sequence of SEQ ID NO:33 as a primer set. Further, to obtain a standard curve for calculating the amount of mRNA expressed, further, PCR was carried out under the same conditions, using the rat genomic DNA (manufactured by Clontech) as a template and the above primer set. For comparison of the individual groups in terms of the amount of the expressed mRNA of the rat MDTS9 gene per given amount of the total RNA, the amount of the expressed mRNA of the rat MDTS9 gene under individual conditions is shown as its ratio to the amount of the expressed G3PDH gene under the individual condition. As a result, it was found that the expressed mRNA of the rat MDTS9 gene in the 5/6 nephrectomized rats was approximately 5-fold of that in the sham-operated rats on 1 week after operation, and was approximately 2-fold on 3 weeks after operation when the amount of urine protein increased, on 6 weeks after operation when the kidney weight began to increase in comparison with the normal kidney weight, and 8 weeks after operation when the diseased conditions were further exacerbated.

### (3) Variation of MDTS9 gene expression in murine 5/6 nephrectomy model

For the murine 5/6 nephrectomy model, the template cDNA was prepared in the same manner as in Reference Example 6(1), and then, the mRNA of the counterpart of rat MDTS9 was assayed in the same manner as in Reference Example 6(2). Under observation of proteinuria and pathohistological sections, the gene expression about 4-fold (the increase of the expression of the gene) was observed in such mouse diagnosed as a severe case.

This Reference Example showed that the expression of the MDTS9 gene was induced in the renal failure models.

### Reference Example 7: Immunohistochemical staining of human renal tissue section

### (1) Preparation of anti-human MDTS9 antibody

According to a laboratory manual [Masato Okada and Kaoru Miyazaki, "Kaitei, *Tanpakushitsu Jikken Noto,* Jyo (*Notebook for Protein Experiments,* Vol.1, revised edition)", Yodo-sha, p.162-79], a fusion protein (GST-MDTS9A) of a peptide consisting of the 280th to 410th amino acids in the amino acid sequence of SEQ ID NO:2 and glutathione S-transferase (GST) was produced in an inclusion body fraction, using a pGEX-6P-1 plasmid (manufactured by Amersham Pharmacia Biotech) as an expression vector and *E*. *coli*. A preparative SDS-polyacrylamide gel electrophoresis (PAGE) was carried out on the inclusion body fraction, to extract the intended GST-MDTS9A protein from the gel by a diffusion method [Masato Okada and Kaoru Miyazaki, "Kaitei, *Tanpakushitsu Jikken Noto*, Ge (*Notebook for Protein Experiments,* Vol.2, revised edition)", Yodo-sha, p.48-51].

The resulting GST-MDTS9A protein was used to immunize a rabbit (Japanese White species) at an interval of 10 to 14 days, five times in total, to prepare an antiserum. The IgG fraction was purified from the antiserum by affinity chromatography using a protein G Sepharose FF column (manufactured by Amersham Pharmacia Biotech). Then, continuously, the resulting purified fraction was purified by affinity chromatography using a column (MBP-MDTS9A column) immobilized with a fusion protein (MBP-MDTS9A) of a peptide consisting of the 280th to 410th amino acids in the amino acid sequence of SEQ ID NO:2 and mannose-bound protein (MBP), to prepare an anti-human MDTS9 antibody. The affinity purification on the protein G Sepharose FF column, the immobilization of MBP-MDTS9A onto a CNBr-activated Sepharose FF column (manufactured by Amersham Pharmacia Biotech), and the affinity purification on the MBP-MDTS9A column were carried out in accordance with the protocols attached thereto. Further, the preparation of MBP-MDTS9A in *E. coli* and the purification thereof were carried out using pMAL-c2E (manufactured by New England Biolabs) as an expression vector and according to an instruction designated as "pMAL Protein Fusion and Purification System" published by the company.

### (2) Detection of MDTS9 protein in human kidney

The anti-human MDTS9 antibody prepared in Reference Example 7(1) reacted with a tissue section fixed with formalin and embedded in paraffin on a slide glass. Then, the tissue section was stained using a commercially available staining kit (VECTORSTAIN ABC-AP kit, catalog No. AK-5000; manufactured by VECTOR LABORATORIES) in accordance with the protocol attached thereto. In this procedure, an anti-rabbit antibody labeled with biotin (Catalog No. BA-1000; manufactured by Vector) as a second antibody and an alkaline phosphatase substrate kit I (Catalog No. SK-5100; manufactured by Vector) as a luminescent substrate were used. As a result, it was observed that the epithelial cells, particularly podocytes of the kidneys of a healthy person and a patient with diabetic nephropathy (at the early stage or latter stage) were stained.

It is apparent from the Reference Example that the MDTS9 protein is expressed in human kidney.

### Example 1: Cloning of 5' upstream region of MDTS9 gene

Using the human genome DNA (Genomic DNA; Clontech) as a template, oligo DNAs of SQ ID Nos.18 and 19 as primers, and PyroBest™ polymerase (Takara Shuzo Co., Ltd.) as a DNA polymerase, PCR was carried out at 97°C for 3 minutes and subsequently by repeating a cycle of treatments at 96.5°C for 30 seconds and 72°C for 2 minutes 5 times, continuously repeating a cycle of treatments at 96.5°C for 30 seconds and 70°C for 2 minutes 5 times, a cycle of treatments at 96.5°C for 30 seconds and 68°C for 2 minutes 5 times, and continuously a cycle of treatments at 96.5°C for 30 seconds, 63°C for 25 seconds and 72°C for 2 minutes 25 times and then under a condition of 72°C for 7 minutes. The resulting fragment of about 2 kbp was subcloned in the *EcoR*V recognition site of pZErO™-2.1 (Invitrogen). The nucleotide sequence of the subclone thus obtained was confirmed. Consequently, the nucleotide sequence was confirmed as a sequence in the 5' upstream region of the MTDS9 gene and was the gene of a nucleotide sequence of the 3253rd to 5023rd nucleotides in SEQ ID NO:17. Using the subclone as a template and oligo DNAs of SEQ ID Nos. 20 and 21 as primers, PCR carried out with PyroBest™ polymerase (Takara Shuzo Co., Ltd.) at 95°C for 3 minutes and subsequently by repeating a cycle of treatments at 97°C for 20 seconds, 59°C for 30 seconds and 72°C for 2 minutes 35 times, and continuously under a condition of 72°C for 3 minutes. A fragment of about 1.8 kbp was generated, where a restriction enzyme *Sac*I recognition sequence was inserted in the 5' side and a restriction enzyme *Hin*dIII recognition sequence was inserted in the 3' side. The DNA fragment was treated with restriction enzymes of *Sac*I and *Hin*dIII, and then inserted in between the *Sac*I and *Hin*dIII sites of a vector for luciferase assay system (PicaGene Vector 2 basic vector; Toyo Ink), to obtain pGV-B2-MDTS9pro2k.

Using a set of the oligo DNAs of SEQ ID NOs:22 and 23, where an *Mlu*I recognition sequence was added to the 5' sides, and a set of the oligo DNAs of SEQ ID Nos. 24 and 25 as primers and the human genome DNA (Genomic DNA; Clontech) as a template, PCR with PyroBest™ DNA polymerase (Takara Shuzo Co., Ltd.) was carried out at 96°C for 2 minutes and subsequently by repeating a cycle of treatments at 97°C for 20 seconds, 60°C for 30 seconds and 72°C for one minute 40 times, and then under a condition of 72°C for 7 minutes. The resulting fragments of about 600 bp and about 1 kbp thus generated were subcloned in the pCR4Blunt-TOPO (Invitrogen), to obtain subclones pCR4Blunt-TOPO5k-1 and pCR4Blunt-TOPO5k-3. Using a set of oligo DNAs SEQ ID Nos. 26 and 27 and a set of oligo DNAs of SEQ ID Nos. 28 and 29 as primers and the human genome DNA (Genomic DNA; Clontech) as a template, PCR with FyroBest™ DNA polymerase (Takara Shuzo Co., Ltd.) was carried out at 96°C for 2 minutes and subsequently by repeating a cycle of treatments at 97°C for 20 seconds, 60°C for 30 seconds and 72°C for 2 minutes 40 times, and continuously under a condition of 72°C for 7 minutes. The resulting fragments of about 1.6 kbp and 1.9 kbp were subcloned in the pCR4Blunt-TOPO (Invitrogen), to obtain subclones pCR4Blunt-TOPO5k-2 and pCR4Blunt-TOPO5k-4. pCR4Blunt-TOPO5k-3 was treated with *Kpn*I and *Sma*I to generate a fragment of about 0.5 kbp while pCR4Blunt-TOPO5k-4 was treated with *Sma*I and *EcoR*I to generate a fragment of about 1.5 kbp. These fragments were conjugated together and inserted in between the *Kpn*I and *EcoR*I sites of pCR4Blunt-TOPO (Invitrogen) to obtain pCR4Blunt-TOPO5k-5. A fragment of about 0.4 kbp obtained from the treatment of pCR4Blunt-TOPO5k-1 with *Mlu*I and *Sph*I, a fragment of about 1.5 kbp obtained from the treatment of pCR4Blunt-TOPO5k-2 with *Sph*I and *Kpn*I, and a fragment of about 2.1 kbp obtained from the treatment of pCR4Blunt-TOPO5k-5 with *Kpn*I and *EcoR*I were conjugated together and inserted in between the *Mlu*I and *EcoR*I sites of pGV-B2-MDTS9pro2k, to obtain pGV-B2-MDTS9pro5k. It was confirmed that the nucleotide sequence of the subclone thus obtained corresponded to the gene of SEQ ID NO:17.

### Example 2: Analysis of DNA sequence in MDTS9 promoter region

The plasmid pGV-B2-MDTS9pro2k or pGV-B2-MDTS9pro5k obtained in Example 1 was introduced in the HEK293-EBNA cell by the method described in Reference Example 3, and cultured in DMEM (GIBCO-BRL), 10% fetal bovine serum, 100 µg/mL penicillin, 100 µg/mL streptomycin, and 250 µg/mL G418 (manufactured by Nakarai Tesque, Inc.), continuously for 48 hours. Then, the luciferase activity was assayed with PicaGene luminescence kit (Toyo Ink). In that case, the assay value was corrected on the basis of the activity value of β-gal expressed with a β-gal expression plasmid (pCH 110 manufactured by Amersham Pharmacia Biotech.) simultaneously introduced. The β-gal activity was assayed, using the Galacto-Light Plus kit (TROPIX). Consequently, apparent increases of the luciferase activity such as about 27-fold in pGV-B2-MDTS9pro2k and about 27 to 40-fold in pGV-B2-MDTS9pro5k, were observed, which were never observed in the original plasmid pGV-B2. This indicates that the promoter activity exists in the DNA fragment.

### Example 3: Response of DNA sequence in MDTS9 promoter region to TGF-β and IL-1

The pGV-B2-MDTS9pro5k obtained in Example 1 was introduced in the HEK293-EBNA cell by the method described in Reference Example 3, and cultured for 16 hours, followed by washing twice with PBS. Subsequently, the culture medium was replaced with DMEM (GIBCO-BRL), 100 µg/mL penicillin, 100 µg/mL streptomycin, and 250 µg/mL G418 (manufactured by Nakarai Tesque, Inc.), for culturing for 6 hours (serum-free culture hereinbelow). Continuously, then, TGF-β (manufactured by Sigma) or IL-1 (manufactured by Sigma) was added and the luciferase activity was assayed after culturing for 24 hours. As in Example 2, the assay value was corrected on the basis of the activity value of β-gal. Consequently, an about 1.7-fold increase of the luciferase activity was observed by the addition of TGF-β (1 ng/ml), while an about 0.7-fold or less decrease of the luciferase activity was observed, by the addition of IL-1 (1 ng/ml) (Figs. 1 and 2). This indicates that a TGF-β response region and an IL-1 response region exist in the DNA fragment. Meanwhile, the luciferase activity in the cell in which pGV-B2-MDTS9pro2k was introduced was also assayed by adding TGF-β to the cell. The resulting luciferase activity was at the same level as in the control.

### INDUSTRIAL APPLICABILITY

The promoter of the present invention is the promoter for a protease MDTS9 in which the gene expression thereof is increased in the renal failure model animals, which is induced by TGF-β but inhibited by IL-1, which is involved in the metabolism of extracellular matrix and which is expressed in human kidney. Therefore, a substance inhibiting the activity of the promoter of the present invention suppresses or inhibits just some of the physiological functions of TGF-β, which are involved in the qualitative change and quantitative increase of extracellular matrix components. Accordingly, such substance is useful as an agent for treating and/or preventing chronic renal failure potentially involving long-term pharmaceutical administration. Thus, the polynucleotide as the promoter of the present invention, and the expression vector and cell therefor are useful as tools for screening an agent for treating and/or preventing chronic renal failure. Using the polynucleotide as the promoter of the present invention, and the expression vector and cell therefor, an agent for treating and/or preventing chronic renal failure can be screened. Additionally, a pharmaceutical composition for the treatment and/or prevention of chronic renal failure can be produced by a method including an analysis step about the presence or absence of the inhibition of the activity of the protease of the invention at a confirmation test according to the quality standard of pharmaceutical compositions for the therapeutic treatment and/or prophylaxis of chronic renal failure and a formulation step.

### FREE TEXT IN SEQUENCE LISTING

Explanation of "Artificial Sequence" are described in the numeric identifier <223> in the Sequence Listing. More particularly, each of the nucleotide sequences of SEQ ID NOS: 3 and 4 is an artificially synthesized linker sequence. Each of the nucleotide sequences of SEQ ID Nos. 5 to 9, 13 and 14 and 20 to 22 is an artificially synthesized primer sequence. The amino acid sequence of SEQ ID NO: 11 is an amino acid sequence obtained by the expression of DNA containing a restriction enzyme NotI recognition nucleotide sequence and a nucleotide sequence encoding the amino acid sequence of the FLAG tag.

As described above, the present invention is explained with reference to specific embodiments, but modifications and improvements thereof obvious to those skilled in the art are also encompassed within the scope of the present invention.

## Claims

1. A polynucleotide having a promoter activity for the polypeptide of SEQ ID NO:2 and comprising a nucleotide sequence consisting of a 3253rd to 5023rd nucleotides in SEQ ID NO:17 or a nucleotide sequence consisting of the 3253rd to the 5023rd nucleotides in SEQ ID NO:17 in which 1 to 10 nucleotides are substituted, deleted, and/or inserted.

2. A polynucleotide according to claim 1, comprising a nucleotide sequence of SEQ ID NO:17 or a nucleotide sequence consisting of a nucleotide sequence of SEQ ID NO:17 in which 1 to 10 nucleotides are substituted, deleted, and/or inserted.

3. A polynucleotide according to claim 1, comprising the nucleotide sequence consisting of the 3253rd to 5023rd nucleotides in SEQ ID NO:17 or the nucleotide sequence of SEQ ID NO:17.

4. An expression vector comprising the polynucleotide according to any one of claims 1 to 3.

5. A cell transfected with the expression vector according to claim 4.

6. A screening tool for a substance for the treatment and/or prevention of chronic renal failure, comprising the polynucleotide according to claim 1 or the cell according to claim 5.

7. Use of the polynucleotide according to claim 1 or the cell according to claim 5 for screening a substance for the treatment and/or prevention of chronic renal failure.

8. A method for analyzing a test compound about whether or not the test compound inhibits the promoter activity of the polynucleotide according to any one of claims 1 to 3, comprising (1) a step of allowing the test compound to contact with the cell according to claim 5 and (2) a step of detecting the promoter activity.

9. A method for screening a substance suppressing the expression of a polypeptide of SEQ ID NO:2, comprising the analyzing step by the method according to claim 8 and a step of selecting a substance inhibiting the promoter activity.

10. A method for screening a substance for the treatment and/or prevention of chronic renal failure by the method according to claim 9.

11. A method for producing a pharmaceutical composition for the treatment and/or prevention of chronic renal failure, comprising the analyzing step by the method according to claim 8 and a formulation step.


SEQUENCE LISTING

<110> Yamanouchi Pharmaceutical Co., Ltd.

<120> Novel promoter

<130> Y0322-PCT

<150> JP2002-180543
<151> 2002-06-20

<160> 33

<170> PatentIn version 3.1

<210> 1
<211> 3675
<212> DNA
<213> Homo sapiens

<220>
<221> CDS
<222> (1)..(3675)

<400> 1
atg aag ccc cgc gcg cgc gga tgg cgg ggc ttg gcg gcg ctg tgg atg 48
Met Lys Pro Arg Ala Arg Gly Trp Arg Gly Leu Ala Ala Leu Trp Met
1               5                   10                  15

ctg ttg gcg cag gtg gcc gag cag gca cct gcg tgc gcc atg gga ccc 96
Leu Leu Ala Gln Val Ala Glu Gln Ala Pro Ala Cys Ala Met Gly Pro
            20                  25                  30

gca gcg gca gcg cct ggg agc ccg agc gtc ccg cgt cct cct cca ccc 144
Ala Ala Ala Ala Pro Gly Ser Pro Ser Val Pro Arg Pro Pro Pro Pro
        35                  40                  45

gcg gag cgg ccg ggc tgg atg gaa aag ggc gaa tat gac ctg gtc tct    192
Ala Glu Arg Pro Gly Trp Met Glu Lys Gly Glu Tyr Asp Leu Val Ser
    50                  55                  60

gcc tac gag gtt gac cac agg ggc gat tac gtg tcc cat gaa atc atg    240
Ala Tyr Glu Val Asp His Arg Gly Asp Tyr Val Ser His Glu Ile Met
65                  70                  75                  80

cac cat cag cgg cgg aga aga gca gtg gcc gtg tcc gag gtt gag tct    288
His His Gln Arg Arg Arg Arg Ala Val Ala Val Ser Glu Val Glu Ser
                85                  90                  95

ctt cac ctt cgg ctg aaa ggc tcc agg cac gac ttc cac gtg gat ctg    336
Leu His Leu Arg Leu Lys Gly Ser Arg His Asp Phe His Val Asp Leu
            100                 105                 110

agg act tcc agc agc cta gtg gct cct ggc ttt att gtg cag acg ttg    384
Arg Thr Ser Ser Ser Leu Val Ala Pro Gly Phe Ile Val Gln Thr Leu
        115                 120                 125

gga aag aca ggc act aag tct gtg cag act tta ccg cca gag gac ttc    432
Gly Lys Thr Gly Thr Lys Ser Val Gln Thr Leu Pro Pro Glu Asp Phe
    130                 135                 140

tgt ttc tat caa ggc tct ttg cga tca cac aga aac tcc tca gtg gcc    480
Cys Phe Tyr Gln Gly Ser Leu Arg Ser His Arg Asn Ser Ser Val Ala
145                 150                 155                 160

ctt tca acc tgc caa ggc ttg tca ggc atg ata cga aca gaa gag gca    528
Leu Ser Thr Cys Gln Gly Leu Ser Gly Met Ile Arg Thr Glu Glu Ala
                165                 170                 175

gat tac ttc cta agg cca ctt cct tca cac ctc tca tgg aaa ctc ggc    576
Asp Tyr Phe Leu Arg Pro Leu Pro Ser His Leu Ser Trp Lys Leu Gly
            180                 185                 190

aga gct gcc caa ggc agc tcg cca tcc cac gta ctg tac aag aga tcc 624
Arg Ala Ala Gln Gly Ser Ser Pro Ser His Val Leu Tyr Lys Arg Ser
        195                 200                 205

aca gag ccc cat gct cct ggg gcc agt gag gtc ctg gtg acc tca agg 672
Thr Glu Pro His Ala Pro Gly Ala Ser Glu Val Leu Val Thr Ser Arg
    210                 215                 220

aca tgg gag ctg gca cat caa ccc ctg cac agc agc gac ctt cgc ctg 720
Thr Trp Glu Leu Ala His Gln Pro Leu His Ser Ser Asp Leu Arg Leu
225                 230                 235                 240

gga ctg cca caa aag cag cat ttc tgt gga aga cgc aag aaa tac atg 768
Gly Leu Pro Gln Lys Gln His Phe Cys Gly Arg Arg Lys Lys Tyr Met
                245                 250                 255

ccc cag cct ccc aag gaa gac ctc ttc atc ttg cca gat gag tat aag 816
Pro Gln Pro Pro Lys Glu Asp Leu Phe Ile Leu Pro Asp Glu Tyr Lys
            260                 265                 270

tct tgc tta cgg cat aag cgc tct ctt ctg agg tcc cat aga aat gaa 864
Ser Cys Leu Arg His Lys Arg Ser Leu Leu Arg Ser His Arg Asn Glu
        275                 280                 285

gaa ctg aac gtg gag acc ttg gtg gtg gtc gac aaa aag atg atg caa 912
Glu Leu Asn Val Glu Thr Leu Val Val Val Asp Lys Lys Met Met Gln
    290                 295                 300

aac cat ggc cat gaa aat atc acc acc tac gtg ctc acg ata ctc aac 960
Asn His Gly His Glu Asn Ile Thr Thr Tyr Val Leu Thr Ile Leu Asn
305                 310                 315                 320

atg gta tct gct tta ttc aaa gat gga aca ata gga gga aac atc aac 1008
Met Val Ser Ala Leu Phe Lys Asp Gly Thr Ile Gly Gly Asn Ile Asn
                325                 330                 335

att gca att gta ggt ctg att ctt cta gaa gat gaa cag cca gga ctg 1056
Ile Ala Ile Val Gly Leu Ile Leu Leu Glu Asp Glu Gln Pro Gly Leu
            340                 345                 350

gtg ata agt cac cac gca gac cac acc tta agt agc ttc tgc cag tgg 1104
Val Ile Ser His His Ala Asp His Thr Leu Ser Ser Phe Cys Gln Trp
        355                 360                 365

cag tct gga ttg atg ggg aaa gat ggg act cgt cat gac cac gcc atc 1152
Gln Ser Gly Leu Met Gly Lys Asp Gly Thr Arg His Asp His Ala Ile
    370                 375                 380

tta ctg act ggt ctg gat ata tgt tcc tgg aag aat gag ccc tgt gac 1200
Leu Leu Thr Gly Leu Asp Ile Cys Ser Trp Lys Asn Glu Pro Cys Asp
385                 390                 395                 400

act ttg gga ttt gca ccc ata agt gga atg tgt agt aaa tat cgc agc 1248
Thr Leu Gly Phe Ala Pro Ile Ser Gly Met Cys Ser Lys Tyr Arg Ser
                405                 410                 415

tgc acg att aat gaa gat aca ggt ctt gga ctg gcc ttc acc att gcc 1296
Cys Thr Ile Asn Glu Asp Thr Gly Leu Gly Leu Ala Phe Thr Ile Ala
            420                 425                 430

cat gag tct gga cac aac ttt ggc atg att cat gat gga gaa ggg aac 1344
His Glu Ser Gly His Asn Phe Gly Met Ile His Asp Gly Glu Gly Asn
        435                 440                 445

atg tgt aaa aag tcc gag ggc aac atc atg tcc cct aca ttg gca gga 1392
Met Cys Lys Lys Ser Glu Gly Asn Ile Met Ser Pro Thr Leu Ala Gly
    450                 455                 460

cgc aat gga gtc ttc tcc tgg tca ccc tgc agc cgc cag tat cta cac 1440
Arg Asn Gly Val Phe Ser Trp Ser Pro Cys Ser Arg Gln Tyr Leu His
465                 470                 475                 480

aaa ttt cta agc acc gct caa gct atc tgc ctt gct gat cag cca aag 1488
Lys Phe Leu Ser Thr Ala Gln Ala Ile Cys Leu Ala Asp Gln Pro Lys
                485                 490                 495

cct gtg aag gaa tac aag tat cct gag aaa ttg cca gga gaa tta tat 1536
Pro Val Lys Glu Tyr Lys Tyr Pro Glu Lys Leu Pro Gly Glu Leu Tyr
            500                 505                 510

gat gca aac aca cag tgc aag tgg cag ttc gga gag aaa gcc aag ctc 1584
Asp Ala Asn Thr Gln Cys Lys Trp Gln Phe Gly Glu Lys Ala Lys Leu
        515                 520                 525

tgc atg ctg gac ttt aaa aag gac atc tgt aaa gcc ctg tgg tgc cat 1632
Cys Met Leu Asp Phe Lys Lys Asp Ile Cys Lys Ala Leu Trp Cys His
    530                 535                 540

cgt att gga agg aaa tgt gag act aaa ttt atg cca gca gca gaa ggc 1680
Arg Ile Gly Arg Lys Cys Glu Thr Lys Phe Met Pro Ala Ala Glu Gly
545                 550                 555                 560

aca att tgt ggg cat gac atg tgg tgc cgg gga gga cag tgt gtg aaa 1728
Thr Ile Cys Gly His Asp Met Trp Cys Arg Gly Gly Gln Cys Val Lys
                565                 570                 575

tat ggt gat gaa ggc ccc aag ccc acc cat ggc cac tgg tcg gac tgg 1776
Tyr Gly Asp Glu Gly Pro Lys Pro Thr His Gly His Trp Ser Asp Trp
            580                 585                 590

tct tct tgg tcc cca tgc tcc agg acc tgc gga ggg gga gta tct cat 1824
Ser Ser Trp Ser Pro Cys Ser Arg Thr Cys Gly Gly Gly Val Ser His
        595                 600                 605

agg agt cgc ctc tgc acc aac ccc aag cca tcg cat gga ggg aag ttc 1872
Arg Ser Arg Leu Cys Thr Asn Pro Lys Pro Ser His Gly Gly Lys Phe
    610                 615                 620

tgt gag ggc tcc act cgc act ctg aag ctc tgc aac agt cag aaa tgt 1920
Cys Glu Gly Ser Thr Arg Thr Leu Lys Leu Cys Asn Ser Gln Lys Cys
625                 630                 635                 640

ccc cgg gac agt gtt gac ttc cgt gct gct cag tgt gcc gag cac aac 1968
Pro Arg Asp Ser Val Asp Phe Arg Ala Ala Gln Cys Ala Glu His Asn
                645                 650                 655

agc aga cga ttc aga ggg cgg cac tac aag tgg aag cct tac act caa 2016
Ser Arg Arg Phe Arg Gly Arg His Tyr Lys Trp Lys Pro Tyr Thr Gln
            660                 665                 670

gta gaa gat cag gac tta tgc aaa ctc tac tgt atc gca gaa gga ttt 2064
Val Glu Asp Gln Asp Leu Cys Lys Leu Tyr Cys Ile Ala Glu Gly Phe
        675                 680                 685

gat ttc ttc ttt tct ttg tca aat aaa gtc aaa gat ggg act cca tgc 2112
Asp Phe Phe Phe Ser Leu Ser Asn Lys Val Lys Asp Gly Thr Pro Cys
    690                 695                 700

tcg gag gat agc cgt aat gtt tgt ata gat ggg ata tgt gag aga gtt 2160
Ser Glu Asp Ser Arg Asn Val Cys Ile Asp Gly Ile Cys Glu Arg Val
705                 710                 715                 720

gga tgt gac aat gtc ctt gga tct gat gct gtt gaa gac gtc tgt ggg 2208
Gly Cys Asp Asn Val Leu Gly Ser Asp Ala Val Glu Asp Val Cys Gly
                725                 730                 735

gtg tgt aac ggg aat aac tca gcc tgc acg att cac agg ggt ctc tac 2256
Val Cys Asn Gly Asn Asn Ser Ala Cys Thr Ile His Arg Gly Leu Tyr
            740                 745                 750

acc aag cac cac cac acc aac cag tat tat cac atg gtc acc att cct 2304
Thr Lys His His His Thr Asn Gln Tyr Tyr His Met Val Thr Ile Pro
        755                 760                 765

tct gga gcc cgg agt atc cgc atc tat gaa atg aac gtc tct acc tcc 2352
Ser Gly Ala Arg Ser Ile Arg Ile Tyr Glu Met Asn Val Ser Thr Ser
    770                 775                 780

tac att tct gtg cgc aat gcc ctc aga agg tac tac ctg aat ggg cac 2400
Tyr Ile Ser Val Arg Asn Ala Leu Arg Arg Tyr Tyr Leu Asn Gly His
785                 790                 795                 800

tgg acc gtg gac tgg ccc ggc cgg tac aaa ttt tcg ggc act act ttc 2448
Trp Thr Val Asp Trp Pro Gly Arg Tyr Lys Phe Ser Gly Thr Thr Phe
                805                 810                 815

gac tac aga cgg tcc tat aat gag ccc gag aac tta atc gct act gga 2496
Asp Tyr Arg Arg Ser Tyr Asn Glu Pro Glu Asn Leu Ile Ala Thr Gly
            820                 825                 830

cca acc aac gag aca ctg att gtg gag ctg ctg ttt cag gga agg aac 2544
Pro Thr Asn Glu Thr Leu Ile Val Glu Leu Leu Phe Gln Gly Arg Asn
        835                 840                 845

ccg ggt gtt gcc tgg gaa tac tcc atg cct cgc ttg ggg acc gag aag 2592
Pro Gly Val Ala Trp Glu Tyr Ser Met Pro Arg Leu Gly Thr Glu Lys
    850                 855                 860

cag ccc cct gcc cag ccc agc tac act tgg gcc atc gtg cgc tct gag 2640
Gln Pro Pro Ala Gln Pro Ser Tyr Thr Trp Ala Ile Val Arg Ser Glu
865                 870                 875                 880

tgc tcc gtg tcc tgc gga ggg gga cag atg acc gtg aga gag ggc tgc 2688
Cys Ser Val Ser Cys Gly Gly Gly Gln Met Thr Val Arg Glu Gly Cys
                885                 890                 895

tac aga gac ctg aag ttt caa gta aat atg tcc ttc tgc aat ccc aag 2736
Tyr Arg Asp Leu Lys Phe Gln Val Asn Met Ser Phe Cys Asn Pro Lys
            900                 905                 910

aca cga cct gtc acg ggg ctg gtg cct tgc aaa gta tct gcc tgt cct 2784
Thr Arg Pro Val Thr Gly Leu Val Pro Cys Lys Val Ser Ala Cys Pro
        915                 920                 925

ccc agc tgg tcc gtg ggg aac tgg agt gcc tgc agt cgg acg tgt ggc 2832
Pro Ser Trp Ser Val Gly Asn Trp Ser Ala Cys Ser Arg Thr Cys Gly
    930                 935                 940

ggg ggt gcc cag agc cgc ccc gtg cag tgc aca cgg cgg gtg cac tat 2880
Gly Gly Ala Gln Ser Arg Pro Val Gln Cys Thr Arg Arg Val His Tyr
945                 950                 955                 960

gac tcg gag cca gtc ccg gcc agc ctg tgc cct cag cct gct ccc tcc 2928
Asp Ser Glu Pro Val Pro Ala Ser Leu Cys Pro Gln Pro Ala Pro Ser
                965                 970                 975

agc agg cag gcc tgc aac tct cag agc tgc cca cct gca tgg agc gcc 2976
Ser Arg Gln Ala Cys Asn Ser Gln Ser Cys Pro Pro Ala Trp Ser Ala
            980                 985                 990

ggg ccc tgg gca gag tgc tca cac acc tgt ggg aag ggg tgg agg aag 3024
Gly Pro Trp Ala Glu Cys Ser His Thr Cys Gly Lys Gly Trp Arg Lys
        995                 1000                1005

cgg gca gtg gcc tgt aag agc acc aac ccc tcg gcc aga gcg cag ctg 3072
Arg Ala Val Ala Cys Lys Ser Thr Asn Pro Ser Ala Arg Ala Gln Leu
    1010                1015                1020

ctg ccc gac gct gtc tgc acc tcc gag ccc aag ccc agg atg cat gaa 3120
Leu Pro Asp Ala Val Cys Thr Ser Glu Pro Lys Pro Arg Met His Glu
1025                1030                1035                1040

gcc tgt ctg ctt cag cgc tgc cac aag ccc aag aag ctg cag tgg ctg 3168
Ala Cys Leu Leu Gln Arg Cys His Lys Pro Lys Lys Leu Gln Trp Leu
                1045                1050                1055

gtg tcc gcc tgg tcc cag tgc tct gtg aca tgt gaa aga gga aca cag    3216
Val Ser Ala Trp Ser Gln Cys Ser Val Thr Cys Glu Arg Gly Thr Gln
            1060                1065                1070

aaa aga ttc tta aaa tgt gct gaa aag tat gtt tct gga aag tat cga    3264
Lys Arg Phe Leu Lys Cys Ala Glu Lys Tyr Val Ser Gly Lys Tyr Arg
        1075                1080                1085

gag ctg gcc tca aag aag tgc tca cat ttg ccg aag ccc agc ctg gag    3312
Glu Leu Ala Ser Lys Lys Cys Ser His Leu Pro Lys Pro Ser Leu Glu
    1090                1095                1100

ctg gaa cgt gcc tgc gcc ccg ctt cca tgc ccc agg cac ccc cca ttt    3360
Leu Glu Arg Ala Cys Ala Pro Leu Pro Cys Pro Arg His Pro Pro Phe
1105                1110                1115                1120

gct gct gcg gga ccc tcg agg ggc agc tgg ttt gcc tca ccc tgg tct    3408
Ala Ala Ala Gly Pro Ser Arg Gly Ser Trp Phe Ala Ser Pro Trp Ser
                1125                1130                1135

cag tgc acg gcc agc tgt ggg gga ggc gtt cag acg agg tcc gtg cag    3456
Gln Cys Thr Ala Ser Cys Gly Gly Gly Val Gln Thr Arg Ser Val Gln
            1140                1145                1150

tgc ctg gct ggg ggc cgg ccg gcc tca ggc tgc ctc ctg cac cag aag    3504
Cys Leu Ala Gly Gly Arg Pro Ala Ser Gly Cys Leu Leu His Gln Lys
        1155                1160                1165

cct tcg gcc tcc ctg gcc tgc aac act cac ttc tgc ccc att gca gag    3552
Pro Ser Ala Ser Leu Ala Cys Asn Thr His Phe Cys Pro Ile Ala Glu
    1170                1175                1180

aag aaa gat gcc ttc tgc aaa gac tac ttc cac tgg tgc tac ctg gta    3600
Lys Lys Asp Ala Phe Cys Lys Asp Tyr Phe His Trp Cys Tyr Leu Val
1185                1190                1195                1200

ccc cag cac ggg atg tgc agc cac aag ttc tac ggc aag cag tgc tgc 3648
Pro Gln His Gly Met Cys Ser His Lys Phe Tyr Gly Lys Gln Cys Cys
                1205                1210                1215

aag act tgc tct aag tcc aac ttg tga 3675
Lys Thr Cys Ser Lys Ser Asn Leu
1220 1225

<210> 2
<211> 1224
<212> PRT
<213> Homo sapiens

<400> 2
Met Lys Pro Arg Ala Arg Gly Trp Arg Gly Leu Ala Ala Leu Trp Met
1               5                   10                  15
Leu Leu Ala Gln Val Ala Glu Gln Ala Pro Ala Cys Ala Met Gly Pro
            20                  25                  30
Ala Ala Ala Ala Pro Gly Ser Pro Ser Val Pro Arg Pro Pro Pro Pro
        35                  40                  45
Ala Glu Arg Pro Gly Trp Met Glu Lys Gly Glu Tyr Asp Leu Val Ser
    50                  55                  60
Ala Tyr Glu Val Asp His Arg Gly Asp Tyr Val Ser His Glu Ile Met
65                  70                  75                  80
His His Gln Arg Arg Arg Arg Ala Val Ala Val Ser Glu Val Glu Ser
                85                  90                  95
Leu His Leu Arg Leu Lys Gly Ser Arg His Asp Phe His Val Asp Leu
            100                 105                 110
Arg Thr Ser Ser Ser Leu Val Ala Pro Gly Phe Ile Val Gln Thr Leu
        115                 120                 125
Gly Lys Thr Gly Thr Lys Ser Val Gln Thr Leu Pro Pro Glu Asp Phe
    130                 135                 140
Cys Phe Tyr Gln Gly Ser Leu Arg Ser His Arg Asn Ser Ser Val Ala
145                 150                 155                 160

Leu Ser Thr Cys Gln Gly Leu Ser Gly Met Ile Arg Thr Glu Glu Ala
                165                 170                 175
Asp Tyr Phe Leu Arg Pro Leu Pro Ser His Leu Ser Trp Lys Leu Gly
            180                 185                 190
Arg Ala Ala Gln Gly Ser Ser Pro Ser His Val Leu Tyr Lys Arg Ser
        195                 200                 205
Thr Glu Pro His Ala Pro Gly Ala Ser Glu Val Leu Val Thr Ser Arg
    210                 215                 220
Thr Trp Glu Leu Ala His Gln Pro Leu His Ser Ser Asp Leu Arg Leu
225                 230                 235                 240
Gly Leu Pro Gln Lys Gln His Phe Cys Gly Arg Arg Lys Lys Tyr Met
                245                 250                 255
Pro Gln Pro Pro Lys Glu Asp Leu Phe Ile Leu Pro Asp Glu Tyr Lys
            260                 265                 270
Ser Cys Leu Arg His Lys Arg Ser Leu Leu Arg Ser His Arg Asn Glu
        275                 280                 285
Glu Leu Asn Val Glu Thr Leu Val Val Val Asp Lys Lys Met Met Gln
    290                 295                 300
Asn His Gly His Glu Asn Ile Thr Thr Tyr Val Leu Thr Ile Leu Asn
305                 310                 315                 320
Met Val Ser Ala Leu Phe Lys Asp Gly Thr Ile Gly Gly Asn Ile Asn
                325                 330                 335
Ile Ala Ile Val Gly Leu Ile Leu Leu Glu Asp Glu Gln Pro Gly Leu
            340                 345                 350
Val Ile Ser His His Ala Asp His Thr Leu Ser Ser Phe Cys Gln Trp
        355                 360                 365
Gln Ser Gly Leu Met Gly Lys Asp Gly Thr Arg His Asp His Ala Ile
    370                 375                 380
Leu Leu Thr Gly Leu Asp Ile Cys Ser Trp Lys Asn Glu Pro Cys Asp
385                 390                 395                 400
Thr Leu Gly Phe Ala Pro Ile Ser Gly Met Cys Ser Lys Tyr Arg Ser
                405                 410                 415
Cys Thr Ile Asn Glu Asp Thr Gly Leu Gly Leu Ala Phe Thr Ile Ala
            420                 425                 430
His Glu Ser Gly His Asn Phe Gly Met Ile His Asp Gly Glu Gly Asn
        435                 440                 445

Met Cys Lys Lys Ser Glu Gly Asn Ile Met Ser Pro Thr Leu Ala Gly
    450                 455                 460
Arg Asn Gly Val Phe Ser Trp Ser Pro Cys Ser Arg Gln Tyr Leu His
465                 470                 475                 480
Lys Phe Leu Ser Thr Ala Gln Ala Ile Cys Leu Ala Asp Gln Pro Lys
                485                 490                 495
Pro Val Lys Glu Tyr Lys Tyr Pro Glu Lys Leu Pro Gly Glu Leu Tyr
            500                 505                 510
Asp Ala Asn Thr Gln Cys Lys Trp Gln Phe Gly Glu Lys Ala Lys Leu
        515                 520                 525
Cys Met Leu Asp Phe Lys Lys Asp Ile Cys Lys Ala Leu Trp Cys His
    530                 535                 540
Arg Ile Gly Arg Lys Cys Glu Thr Lys Phe Met Pro Ala Ala Glu Gly
545                 550                 555                 560
Thr Ile Cys Gly His Asp Met Trp Cys Arg Gly Gly Gln Cys Val Lys
                565                 570                 575
Tyr Gly Asp Glu Gly Pro Lys Pro Thr His Gly His Trp Ser Asp Trp
            580                 585                 590
Ser Ser Trp Ser Pro Cys Ser Arg Thr Cys Gly Gly Gly Val Ser His
        595                 600                 605
Arg Ser Arg Leu Cys Thr Asn Pro Lys Pro Ser His Gly Gly Lys Phe
    610                 615                 620
Cys Glu Gly Ser Thr Arg Thr Leu Lys Leu Cys Asn Ser Gln Lys Cys
625                 630                 635                 640
Pro Arg Asp Ser Val Asp Phe Arg Ala Ala Gln Cys Ala Glu His Asn
                645                 650                 655
Ser Arg Arg Phe Arg Gly Arg His Tyr Lys Trp Lys Pro Tyr Thr Gln
            660                 665                 670
Val Glu Asp Gln Asp Leu Cys Lys Leu Tyr Cys Ile Ala Glu Gly Phe
        675                 680                 685
Asp Phe Phe Phe Ser Leu Ser Asn Lys Val Lys Asp Gly Thr Pro Cys
    690                 695                 700
Ser Glu Asp Ser Arg Asn Val Cys Ile Asp Gly Ile Cys Glu Arg Val
705                 710                 715                 720
Gly Cys Asp Asn Val Leu Gly Ser Asp Ala Val Glu Asp Val Cys Gly
                725                 730                 735

Val Cys Asn Gly Asn Asn Ser Ala Cys Thr Ile His Arg Gly Leu Tyr
            740                 745                 750
Thr Lys His His His Thr Asn Gln Tyr Tyr His Met Val Thr Ile Pro
        755                 760                 765
Ser Gly Ala Arg Ser Ile Arg Ile Tyr Glu Met Asn Val Ser Thr Ser
    770                 775                 780
Tyr Ile Ser Val Arg Asn Ala Leu Arg Arg Tyr Tyr Leu Asn Gly His
785                 790                 795                 800
Trp Thr Val Asp Trp Pro Gly Arg Tyr Lys Phe Ser Gly Thr Thr Phe
                805                 810                 815
Asp Tyr Arg Arg Ser Tyr Asn Glu Pro Glu Asn Leu Ile Ala Thr Gly
            820                 825                 830
Pro Thr Asn Glu Thr Leu Ile Val Glu Leu Leu Phe Gln Gly Arg Asn
        835                 840                 845
Pro Gly Val Ala Trp Glu Tyr Ser Met Pro Arg Leu Gly Thr Glu Lys
    850                 855                 860
Gln Pro Pro Ala Gln Pro Ser Tyr Thr Trp Ala Ile Val Arg Ser Glu
865                 870                 875                 880
Cys Ser Val Ser Cys Gly Gly Gly Gln Met Thr Val Arg Glu Gly Cys
                885                 890                 895
Tyr Arg Asp Leu Lys Phe Gln Val Asn Met Ser Phe Cys Asn Pro Lys
            900                 905                 910
Thr Arg Pro Val Thr Gly Leu Val Pro Cys Lys Val Ser Ala Cys Pro
        915                 920                 925
Pro Ser Trp Ser Val Gly Asn Trp Ser Ala Cys Ser Arg Thr Cys Gly
    930                 935                 940
Gly Gly Ala Gln Ser Arg Pro Val Gln Cys Thr Arg Arg Val His Tyr
945                 950                 955                 960
Asp Ser Glu Pro Val Pro Ala Ser Leu Cys Pro Gln Pro Ala Pro Ser
                965                 970                 975
Ser Arg Gln Ala Cys Asn Ser Gln Ser Cys Pro Pro Ala Trp Ser Ala
            980                 985                 990
Gly Pro Trp Ala Glu Cys Ser His Thr Cys Gly Lys Gly Trp Arg Lys
        995                 1000                1005
Arg Ala Val Ala Cys Lys Ser Thr Asn Pro Ser Ala Arg Ala Gln Leu
    1010                1015                1020

Leu Pro Asp Ala Val Cys Thr Ser Glu Pro Lys Pro Arg Met His Glu
1025                1030                1035                1040
Ala Cys Leu Leu Gln Arg Cys His Lys Pro Lys Lys Leu Gln Trp Leu
                1045                1050                1055
Val Ser Ala Trp Ser Gln Cys Ser Val Thr Cys Glu Arg Gly Thr Gln
            1060                1065                1070
Lys Arg Phe Leu Lys Cys Ala Glu Lys Tyr Val Ser Gly Lys Tyr Arg
        1075                1080                1085
Glu Leu Ala Ser Lys Lys Cys Ser His Leu Pro Lys Pro Ser Leu Glu
    1090                1095                1100
Leu Glu Arg Ala Cys Ala Pro Leu Pro Cys Pro Arg His Pro Pro Phe
1105                1110                1115                1120
Ala Ala Ala Gly Pro Ser Arg Gly Ser Trp Phe Ala Ser Pro Trp Ser
                1125                1130                1135
Gln Cys Thr Ala Ser Cys Gly Gly Gly Val Gln Thr Arg Ser Val Gln
            1140                1145                1150
Cys Leu Ala Gly Gly Arg Pro Ala Ser Gly Cys Leu Leu His Gln Lys
        1155                1160                1165
Pro Ser Ala Ser Leu Ala Cys Asn Thr His Phe Cys Pro Ile Ala Glu
    1170                1175                1180
Lys Lys Asp Ala Phe Cys Lys Asp Tyr Phe His Trp Cys Tyr Leu Val
1185                1190                1195                1200
Pro Gln His Gly Met Cys Ser His Lys Phe Tyr Gly Lys Gln Cys Cys
                1205                1210                1215
Lys Thr Cys Ser Lys Ser Asn Leu
            1220

<210> 3
<211> 50
<212> DNA
<213> Artificial Sequence

<220>
<223> Description of Artificial Sequence: an

artificially synthesized linker sequence

<400> 3
ctagcgcggc cgcaggatcc gactacaagg acgacgatga caaatgataa          50

<210> 4
<211> 50
<212> DNA
<213> Artificial Sequence

<220>
<223> Description of Artificial Sequence: an
      artificially synthesized linker sequence

<400> 4
gatcttatca tttgtcatcg tcgtccttgt agtcggatcc tgcggccgcg          50

<210> 5
<211> 34
<212> DNA
<213> Artificial Sequence

<220>
<223> Description of Artificial Sequence: an
      artificially synthesized primer sequence

<400> 5
ggactagtct agaagctggg taccagctgc tagc                           34

<210> 6
<211> 29
<212> DNA
<213> Artificial Sequence

<220>
<223> Description of Artificial Sequence: an
      artificially synthesized primer sequence

<400> 6
ggactagtgt cgaccggtca tggctgcgc                                          29


<210> 7
<211> 38
<212> DNA
<213> Artificial Sequence

<220>
<223> Description of Artificial Sequence: an
      artificially synthesized primer sequence

<400> 7
ggactagtgc catgggaccc gcagcggcag cgcctggg                                38


<210> 8
<211> 40
<212> DNA
<213> Artificial Sequence

<220>
<223> Description of Artificial Sequence: an
      artificially synthesized primer sequence

<400> 8
gggcggccgc acccctgtga atcgtgcagg ctgagttatt                              40


<210> 9

<211> 41
<212> DNA
<213> Artificial Sequence

<220>
<223> Description of Artificial Sequence: an
      artificially synthesized primer sequence

<400> 9
ggactagtac catgaagccc cgcgcgcgcg gatggcgggg c                41


<210> 10
<211> 30
<212> DNA
<213> Homo sapiens

<400> 10
ccctgtggtc aacctcgtag gcagagacca                             30


<210> 11
<211> 11
<212> PRT
<213> Artificial Sequence

<220>
<223> Description of Artificial Sequence: an amino acid
sequence obtained by expression of a DNA containing a
restriction enzyme NotI recognition nucleotide sequence and a
nucleotide sequence encoding a FLAG tag amino acid sequence

<400> 11
Ala Ala Ala Asp Tyr Lys Asp Asp Asp Asp Lys
 1               5                   10

<210> 12
<211> 5
<212> PRT
<213> Homo sapiens

<400> 12
His Glu Ser Gly His
1               5

<210> 13
<211> 41
<212> DNA
<213> Artificial Sequence

<220>
<223> Description of Artificial Sequence: an
      artificially synthesized primer sequence

<400> 13
tggccttcac cattgcccat cagtctggac acaactttgg c                                41

<210> 14
<211> 41
<212> DNA
<213> Artificial Sequence

<220>
<223> Description of Artificial Sequence: an
      artificially synthesized primer sequence

<400> 14
gccaaagttg tgtccagact gatgggcaat ggtgaaggcc a                                41

<210> 15
<211> 31
<212> DNA
<213> Homo sapiens

<400> 15
caccttaagt agcttctgcc agtggcagtc t 31

<210> 16
<211> 32
<212> DNA
<213> Homo sapiens

<400> 16
acaaacatta cggctatcct ccgagcatgg ag 32

<210> 17
<211> 5023
<212> DNA
<213> Homo sapiens

<400> 17
atatgaacta caaaaatttt gcacacaatc attgtaattt tttccactaa agacaagtgt 60
tgatgcacaa tacaatgcta ccttctttga aacctgacta gtttcagctg tgtgtgagat 120
gaggaaggaa caaaattaca gactaaacag aatctttgta gggcacaaac acatccaatt 180
cttctctgtt tcggttgtat ggatggtggt tttacataca aggtaatgtg cccacttgca 240
ctgctttagt ttaaagttta tgcaatttag aaaagggtta tgacaaagtt tggtcctttt 300
aatctaaaat gccatttgtt tccacatgag aaagggccct ggctcctttc attgtgaaag 360
ggcactgggc atgggagagc atgcagccgt tagaagaaac tctctctcag atcctttgct 420
aagcactttg tttttatttt taatttaatt tttattctct attgaaacgt ctacttgata 480
ttcaaaatga cctaagtaat cagaacctct gtctgaaagg aaacaagtgc taactgtgaa 540
tggtctaaat aatttacagc aacatttttc tgtgaagggc cagatggtaa atagtttagg 600
ctttgcaggg cacacagtct ctgttgcagt gacttaactc tgccattgtg aaagcattca 660

cagacgatct gcaaacaaat aaaactttat ttacaaaatg agtcagctgg ccagatttga 720
tcttaggcag tagagagttt tctgaccctg gatgtaaaca aatgatgaca aaactgcttt 780
cctaaacttt gacctcaaag ttaagagtgc taagttcttg aagggctgca aagtgaagta 840
gtttgaggct ggcggtttgt tttgactctt ccaatatttg ttctcagggt tactttgctg 900
ggacaccttt aaactgaagg gactccatat acatatttag atttccattt tacctttaaa 960
aattaggcag gtctggtccc caatactgta tggcaccaac ccatgcgctg gccactcagt 1020
gccaggagtc ccttttaagg cagacacgtg tgctccagtt tgcccctccc tctccatccc 1080
tccctgagac ggacaggcag ctgcgagtta ctgccaccat gttagatggc cagtcttaaa 1140
ctcggtccac tcgtgtgttt tgcatctcag ctcccactct ggcctctata gcctggactc 1200
aaccacccct ctttgttacc tggagacatt catactgcag gatgagcgct gaaatccgtg 1260
caccgctaga ggaactggga aacactagtc attttaaaga atgttgtttc tttcttcaca 1320
atgaaaagag actcatttag cactgctctg gtcttactgt tcttaagcat accgtaagag 1380
tgaatccttt tatatcagcc attcttcttt ttttccatct gtttgaactt acagaagagg 1440
gcccctaggt atctccatgt gatagactgg aaaaaatctc actccctttc atgttacact 1500
ggacacaatt agaagtacgt agagatcccc atttatcaag cgtgcaatgt actcaggaca 1560
agggaaattc tgccggcaga ttctggagac ttccaggcac tgccgaaccc ccccttcaag 1620
gtaagtgtaa gctccctgag gtgtgagctg aggcagacgc ttgttaggca ttggcagaga 1680
ggaagggtgg caggtgtttt aaggtccctc ccaatgccag gctttatgta aaaaatattt 1740
gcactttgag gaatggtttc aaataaatga ataagagagg gagtgtgtgt tgctatggaa 1800
tgaggatgcc catacacagc caggcggttc tactgcccct acctggggtc tggggcttca 1860
ggacagccct agcagggcgc ctgctgggag ctctccctgg gtacccacag actcgcgaca 1920
ggctgggcaa agaaagccag agcccaagac accatttccc ccattcatcc ccctctccaa 1980
agtgtgcaaa agaggcaacg tcaagctaag ctggctgtga aggacactgg aaccaaacaa 2040
agggcagctg aaggcccagg atccacataa ggtgtgtgat gggaaagcag ccacggatgg 2100
ggagcgccac acacacacac gtgtgcaaac atgcactccc acgcgcgcag tcctactgag 2160
aggtgacagc gtgctggcag tcctcagagc cctcgcttgc tctgggcacc tcccctgcct 2220
gggctccgac tttggcggca tttgaggagc ccttcagctc cccactgcac tgtgggagcc 2280
cctttctggg ctggccaagg ctggagccca ctccctcagc ttgcagggag gtgtggaggg 2340
agaggcacga gcgggaaccg gggctgcgtg cagcgcttgc gggccagctg gagttccggg 2400
tgggcgtggg cttggcgggc cccgcactca gagcagccgg ccagccctgc tggccccggg 2460
caatgaggga cttagcacct gggccagtgg ctgcggaggg tgtactgggt cccccagcag 2520
tgccggccca ccggggctgt gctcgatttc ttgccgagcc ttagctgcct tcctgcgggg 2580
cagggctcgg gacctgcagc ccgccatgcc tgagcctccc acccactcca tgggctcctg 2640
tgcggcccga gcctccccaa ggagcgccac cccctgctcc acagcgccca gtcccatgga 2700
ccacccaagg gctgaggaat gcgagcgcac ggcgcaggac tggcaggcag ctccacctgc 2760
gccccggtgc ggggatccac taggtgaagc cagctgggat cctgagtctg gtggggatgt 2820

ggagagtctt tatgtctagc tcagggattg taaatacacc aatcagcacc ctgtgtttag 2880
ctcaaggttt gtgagtgcac caatcgatac tctgtatcta gctgctctgg tggggccttg 2940
gagaacctgt gtgtggaaac tctgtgtatc taactaatct gatggggacg tggagaacct 3000
ttgtatctag ctcagggatg gtaaacgcac caatcagcgc cctgacaaaa caggccactc 3060
ggctctacca atcagcagga tgtcgctagg gccagataag agaataaaag caggctgccc 3120
cagccagccg tggcaaccgg ctcaggtccc cttccatgct gtggaagctt tgttcttttg 3180
ctttttgcaa taaatcttac tactgctcac tctttttttt tttttttttt tttttttttt 3240
tgagacggag tctcgctctg tcgcccaggc tggagtgcag tggcgggatc tcggctcact 3300
gcaagctccg cctcccgggt tcacgccatt ctcctgcctc agcctcccaa gtagctggga 3360
ctacaggcgc ccgccactac gcccgtctaa ttttttgtat ttttagtaga gacggggttt 3420
caccgtttta gccgggatgg tctcgatctc ctgacctcgt gatccgcccg cctcggcctc 3480
ccaaagtgct gggattacag gcgtgagcca ccgcgcccgg cctactgctc actctttggg 3540
tccactctgc tttcatgagc tgtaacactc accgtgaaga tctgcagctt cactcctgag 3600
cccagcgaga ccacgagccc accgggaaga acgaacaact ccagacgctc tgccttaaga 3660
gctgtaacac tcaccgccta ggtctgcagc ttcaatcctg agccagcgag accacgaacc 3720
caccagaagg aagaaactcc gaacacatct gaacatcaga agtaacaaac tccagacgca 3780
ccaccttaag agctgtaccc actcaccgcg agggtatgcg gcttcattct tgaagtcagt 3840
gagaccaaga acccaccaat tccagacaca ctatgggctc acagcgtgta ctcgcgcaca 3900
cgcagtcagg tgtggatgta cacccgcgca cacccaggca catgtacacc cgcgcgctca 3960
cacaccccat ccagctacag cagaattctg gcgaggctgt tgaccgcaca cctgctgcct 4020
ccttggccac cctgtccaca cagtagcccg atcgaccccc gtggcggccg agacccaggc 4080
ccatccgcag ccctgagacc tccctaggga ttgcacccag cagccagtca ccggcctccg 4140
cggcctggcc agttgagggt ggccgtgacc gcggggccag gagcgccgcc acatctcggg 4200
gcaaatggcg cgggggaaga gtttcctcct cagcctcccc gtctccgatc gctccgcaaa 4260
ctccagagcg aggcacgcgc ctttaaaggc aggtccgcgg ctctcccacg tcctggcgcc 4320
cggttttccg cacccagtgt ccccacagct gtgcccgggc acagaggcgc ggccagaccg 4380
cactccgcgg gctgcaggtg tcccggcctc tggcggcgcc ggtgcggccc ggaggtggga 4440
gcccgcggag ccactgcagt agctggagtc ccgccgagtc cccagcccca aggcagggca 4500
ggagcgcgca ccggccggag gtccatgctg agcatcgccc gcgccggtgc ccggcagcct 4560
ctccaactgt gtggtccccg cgctggcaga gaggcacgga ctgcaggccg tgggcagctc 4620
catcttcccg cgtcctcctc ctctggcgct gcccgctgtc tcccgccttc cctctgctcc 4680
cctctcgcct ccgcctcagc gccccgctga cctcgcctcc tcccctctgc tctttgtccc 4740
tgcactctcc cctcctcggt cctctgaccc ccccgccctc acctcctccc ctcctctctc 4800
ccctgcccgc cccgcgctct cccaccgctc ccgccgcccc cgccgccgcg gctgccactc 4860
cgccccccgc gccgcacgga gcttcagtaa taaccccggc gcggcggcgg agtcgctgtg 4920
gggaatcctc ccgcgctctg cctgggtcgg gtcctccctg cccgctcgca cgctgccggc 4980

cggggaccct ccggtggccc ctagcccctc ggagcgctcc tgg                    5023

<210> 18
<211> 30
<212> DNA
<213> Homo sapiens

<400> 18
tttgagacgg agtctcgctc tgtcgcccag                                    30

<210> 19
<211> 29
<212> DNA
<213> Homo sapiens

<400> 19
ccaggagcgc tccgaggggc taggggcca                                     29

<210> 20
<211> 43
<212> DNA
<213> Artificial Sequence

<220>
<223> Description of Artificial Sequence: an
      artificially synthesized primer sequence

<400> 20
aagagctctg ctagctgaga cggagtctcg ctctgtcgcc cag                     43

<210> 21
<211> 43

<212> DNA
<213> Artificial Sequence

<220>
<223> Description of Artificial Sequence: an
      artificially synthesized primer sequence

<400> 21
taaagcttag atctccagga gcgctccgag gggctagggg cca                43

<210> 22
<211> 42
<212> DNA
<213> Artificial Sequence

<220>
<223> Description of Artificial Sequence: an
      artificially synthesized primer sequence

<400> 22
aaacgcgtat atgaactaca aaaattttgc acacaatcat tg                42

<210> 23
<211> 25
<212> DNA
<213> Homo sapiens

<400> 23
catctggccc ttcacagaaa aatgt                                   25

<210> 24
<211> 24
<212> DNA

<213> Homo sapiens

<400> 24
cctaggtatc tccatgtgat agac                                          24


<210> 25
<211> 30
<212> DNA
<213> Homo sapiens

<400> 25
aaggcagcta aggctcggca agaaatcgag                                    30


<210> 26
<211> 31
<212> DNA
<213> Homo sapiens

<400> 26
gtttggtcct tttaatctaa aatgccattt g                                  31


<210> 27
<211> 25
<212> DNA
<213> Homo sapiens

<400> 27
atggtgtctt gggctctggc tttct                                         25


<210> 28
<211> 31
<212> DNA

<213> Homo sapiens

<400> 28
aacaaagggc agctgaaggc ccaggatcca c                                    31

<210> 29
<211> 28
<212> DNA
<213> Homo sapiens

<400> 29
tggacagggt ggccaaggag gcagcagg                                        28

<210> 30
<211> 19
<212> DNA
<213> Rattus sp.

<400> 30
agcctagctc ccgatccaa                                                  19

<210> 31
<211> 21
<212> DNA
<213> Rattus sp.

<400> 31
ccaccaccag agtctccaca t                                               21

<210> 32
<211> 15
<212> DNA

<213> Rattus sp.

<400> 32
aagcaggcgg ccgag                                                    15

<210> 33
<211> 21
<212> DNA
<213> Rattus sp.

<400> 33
atcaaaggtg gaagaatggg a                                             21